# EUROPEAN PATENT APPLICATION

(11) **EP 4 628 087 A1**
(43) Date of publication of application: **08.10.2025**
(21) Application number: 23907216.8
(22) Date of filing: 25.12.2023
(51) Int. Cl.: A61K 35/744, A61P 31/14, A61P 31/16, C12N 1/20

(54) **PHARMACEUTICAL COMPOSITION FOR TOPICAL ADMINISTRATION**

(30) Priority: 23.12.2022 JP 2022206490
(71) Applicant: Japan Institute for Health Security, Tokyo 162-8655 (JP); KIRIN HOLDINGS KABUSHIKI KAISHA, Nakano-ku, Tokyo 164-0001 (JP)
(72) Inventor: ISHII Hiroshi, Tokyo 162-8655 (JP); MATANO Tetsuro, Tokyo 162-8655 (JP); JONAI Kenta, Tokyo 164-0001 (JP); TSUJI Ryohei, Tokyo 164-0001 (JP)
(74) Representative: Hoffmann Eitle
(86) International application number: PCT/JP2023/046405
(87) International publication number: WO 2024/135856

(57) **Abstract**

The purpose of the present invention is to provide a novel pharmaceutical composition for topical administration and a novel immunostimulating composition for topical administration. The present invention provides a pharmaceutical composition for topical administration and an immunostimulating composition for topical administration, the composition comprising a lactic acid bacterium as an active ingredient. The lactic acid bacterium that is the active ingredient in the present invention is preferably *Lactococcus lactis* subsp. *lactis.* The present invention is advantageous because topical administration (especially intranasal administration) can enhance the prophylactic and immunostimulatory effects of lactic acid bacterium, an active ingredient, on infectious diseases.

## Description

### Reference to Related Application

The present application benefits from the priority of a prior Japanese application, Japanese Patent Application No. 2022-206490 (filing date: Dec. 23, 2022), the disclosure of which is incorporated by reference as a part of this specification in its entirety.

### Technical Field

The present invention relates to a pharmaceutical composition for topical administration.

### Background Art

The worldwide epidemic of infectious diseases caused by viruses or bacteria, including the novel coronavirus (SARS-CoV-2), has become a social problem. As drugs for a novel coronavirus infection caused by SARS-CoV-2 (COVID-19), for example, antibody medicines using neutralizing monoclonal antibodies, etc., have been developed (Non Patent Literatures 1 to 3). On the other hand, there are no prophylactics that are for the novel coronavirus infection and have efficacy and effectiveness approved as pharmaceutical products, and their development has thus been sought. In particular, because of the high likelihood of the emergence of variants of the virus, there is a need to develop therapeutic and prophylactic agents that should be further effective against the variants.

Lactic acid bacteria are abundant in yogurt and other fermented milk products, and have long been used in foods and beverages. In addition, lactic acid bacteria are known to bring about various physiological activities in the intestines, etc., by oral intake, but their effects by topical administration for pharmaceutical use are unknown.

### Citation List

### Non Patent Literature

Non Patent Literature 1: Weinreich DM, et al., N Engl J Med., 2021; 384(3): 238-251.
Non Patent Literature 2: Piccicacco N, et al., Open Forum Infect Dis., 2021; 8(7): ofab292.
Non Patent Literature 3: Koehler J, et al., Infection, 2021; 49(6): 1313-1318.

### Summary of Invention

### Technical Problem

The purpose of the present invention is to provide a novel pharmaceutical composition for topical administration.

### Solution to Problem

The present inventors have found that topical administration of lactic acid bacterium can enhance the prophylactic effect on infectious diseases. The present inventors have also found that topical administration of lactic acid bacterium can elicit an immunostimulatory effect. The present invention is based on these findings.

The present invention provides the following items of the invention.
[1] A pharmaceutical composition for topical administration, comprising a lactic acid bacterium as an active ingredient.
[2] The pharmaceutical composition according to [1] above, wherein the topical administration is intranasal administration, sublingual administration, or inhalation administration.
[3] The pharmaceutical composition according to [1] or [2] above, wherein the topical administration is administration to an upper and/or lower respiratory tract.
[4] The pharmaceutical composition according to [3] above, wherein the upper respiratory tract is a nasal cavity.
[5] The pharmaceutical composition according to any one of [1] to [4] above for inducing production of an interferon by plasmacytoid dendritic cells in an upper and/or lower respiratory tract.
[6] The pharmaceutical composition according to [5] above, wherein after a subject of administration of the pharmaceutical composition contracts an infectious disease, the production of IFN is induced in a subject.
[7] The pharmaceutical composition according to any one of [1] to [6] above for prevention or treatment of an infectious disease or for use in reducing a risk of contracting an infectious disease in a subject of administration.
[8] The pharmaceutical composition according to [7] above, wherein the infectious disease is a viral infectious disease.
[9] The pharmaceutical composition according to [8] above, wherein the prevention of the viral infectious disease is prevention of onset of the viral infectious disease or prevention of aggravation of the viral infectious disease.
[10] The pharmaceutical composition according to [8] or [9] above, wherein the viral infectious disease is COVID-19 or an influenza virus infection.
[11] The pharmaceutical composition according to any one of [8] to [10] above, wherein the viral infectious disease is an infection caused by a respiratory infection-causing virus.
[12] The pharmaceutical composition according to [11] above, wherein the respiratory viral infection-causing virus is SARS-CoV-2 or an influenza virus.
[13] The pharmaceutical composition according to any one of [1] to [12] above, which is administered to a mammal.
[14] The pharmaceutical composition according to [13] above, wherein the mammal is a human.
[15] The pharmaceutical composition according to any one of [1] to [14] above, wherein the number of doses is two or more.
[16] The pharmaceutical composition according to any one of [1] to [15] above, wherein a dosing interval is one day or more.
[17] The pharmaceutical composition according to any one of [8] to [16] above, wherein an effective period of the prevention of the viral infectious disease is 56 days from a last day of the topical administration.
[18] The pharmaceutical composition according to any one of [1] to [17] above, wherein a daily dose (based on 50 kg body weight of an adult) of the lactic acid bacterium is 1 mg or more and 1000 mg or less.
[19] The pharmaceutical composition according to any one of [1] to [18] above, wherein the lactic acid bacterium is *Lactococcus lactis* subsp. *lactis* JCM5805 strain.
[20] A composition for topical administration, comprising *Lactococcus lactis* subsp. *lactis* as an active ingredient.
[21] The composition for topical administration according to [20] above, which is a pharmaceutical composition.
[22] The pharmaceutical composition according to any one of [8] to [19] above, which is topically administered at a site of viral infection.
[23] An immunostimulating composition for topical administration, comprising a lactic acid bacterium as an active ingredient.
[24] The immunostimulating composition according to [22] or [23] above for enhancing expression of IFN-inducible antiviral gene (ISG) in a submandibular lymph node and/or a spleen.
[25] The immunostimulating composition according to [24] above, wherein the ISG is one or two or more kinds selected from the group consisting of Viperin, Isg15, and Mx1.
[26] The immunostimulating composition according to any one of [22] to [25] above for increasing a ratio of pDC to lymphocytes in a spleen and/or a ratio of pDC to lymphocytes in nasal mucosa.
[27] The immunostimulating composition according to any one of [22] to [26] above for increasing a ratio of CD11b⁺Siglec-H⁺ cells to lymphocytes in nasal mucosa.
[28] A method of preventing or a method of treating an infectious disease, a method of reducing a risk of contracting an infectious disease, or a method of immunostimulation, the method comprising the step of topically administering an effective amount of lactic acid bacterium or a composition comprising the lactic acid bacterium to a subject in need thereof.
[29] Use of a lactic acid bacterium for the manufacture of an infectious disease prophylactic or therapeutic agent for topical administration, for the manufacture of an infectious disease contraction risk reducing agent for topical administration, or for the manufacture of an immunostimulating agent for topical administration, or as an infectious disease prophylactic or therapeutic agent for topical administration, as an infectious disease contraction risk reducing agent for topical administration or an immunostimulating agent for topical administration, or in a method of preventing or treating an infectious disease by topical administration, in a method of reducing a risk of contracting an infectious disease by topical administration, or in a method of immunostimulation by topical administration.
[30] A lactic acid bacterium for use in prevention or treatment of an infectious disease by topical administration, for use in reducing a risk of contracting an infectious disease by topical administration, or for use in immunostimulation by topical administration.

The present invention is advantageous because topical administration (especially intranasal administration) can enhance the prophylactic and immunostimulatory effects of lactic acid bacterium, an active ingredient, on infectious diseases.

### Brief Description of Drawings

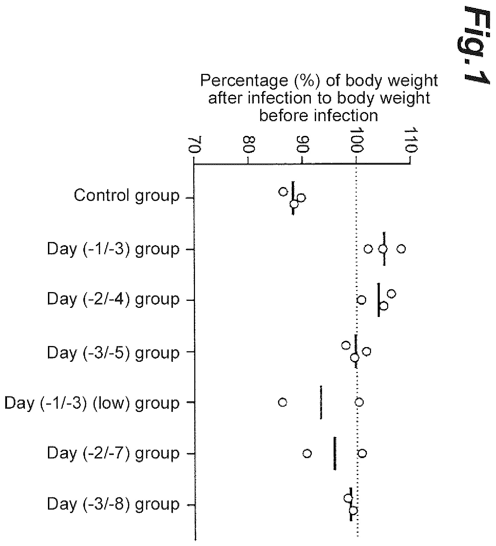

[Fig. 1] Fig. 1 shows the percentage of body weight at 3 days after infection relative to the pre-infection body weight of the control group or the lactic acid bacterium administration groups (6 groups) in the case of intranasal administration.

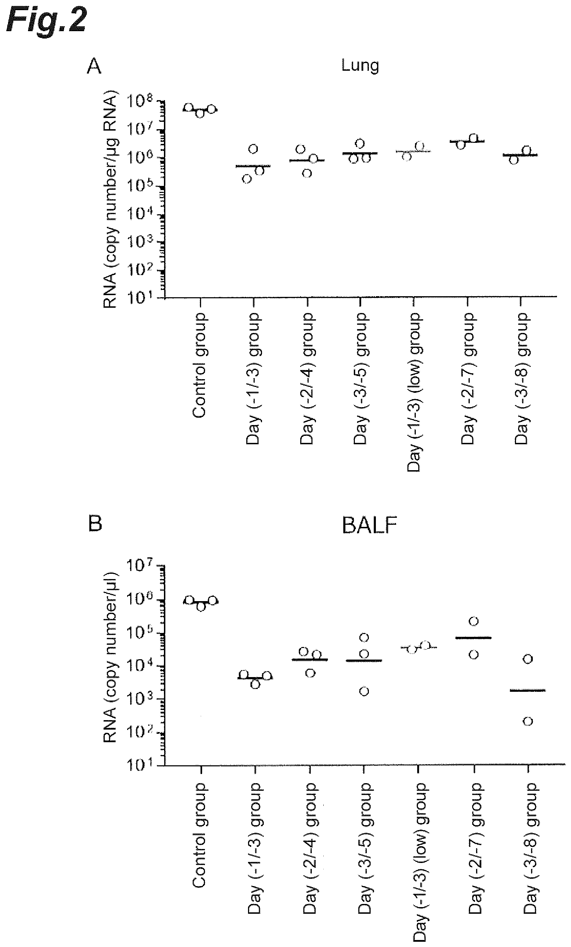

[Fig. 2] Fig. 2A shows the level of SARS-CoV-2 RNA in the lungs of the control group or the lactic acid bacterium administration groups (6 groups) at 3 days after infection in the case of intranasal administration. Fig. 2B shows the level of SARS-CoV-2 RNA in the BALF of the control or the lactic acid bacterium administration groups (6 groups) at 3 days after infection in the case of intranasal administration.

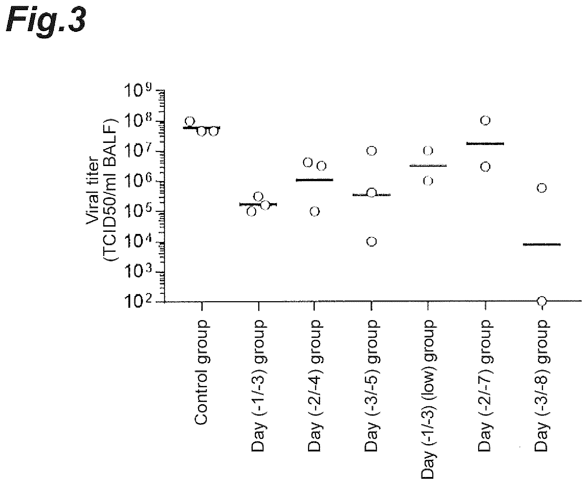

[Fig. 3] Fig. 3 shows the viral titer (viral load) of SARS-CoV-2 in the BALF of the control group or the lactic acid bacterium administration groups (6 groups) at 3 days after infection in the case of intranasal administration.

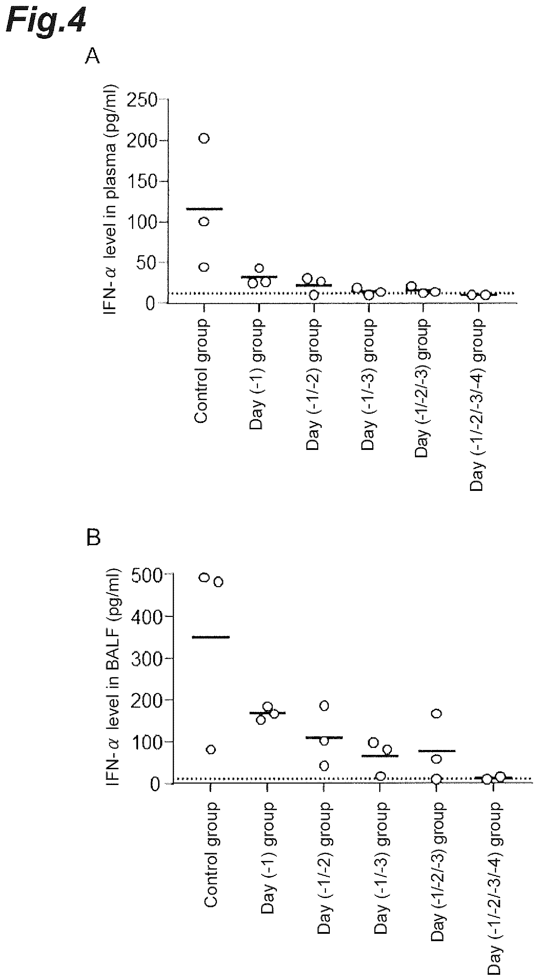

[Fig. 4] Fig. 4A shows the IFN-α level in plasma of the control group or the lactic acid bacterium administration groups (5 groups) at 3 days after infection in the case of intranasal administration. Fig. 4B shows the IFN-α level in BALF of the control group or the lactic acid bacterium administration groups (5 groups) at 3 days after infection in the case of intranasal administration.

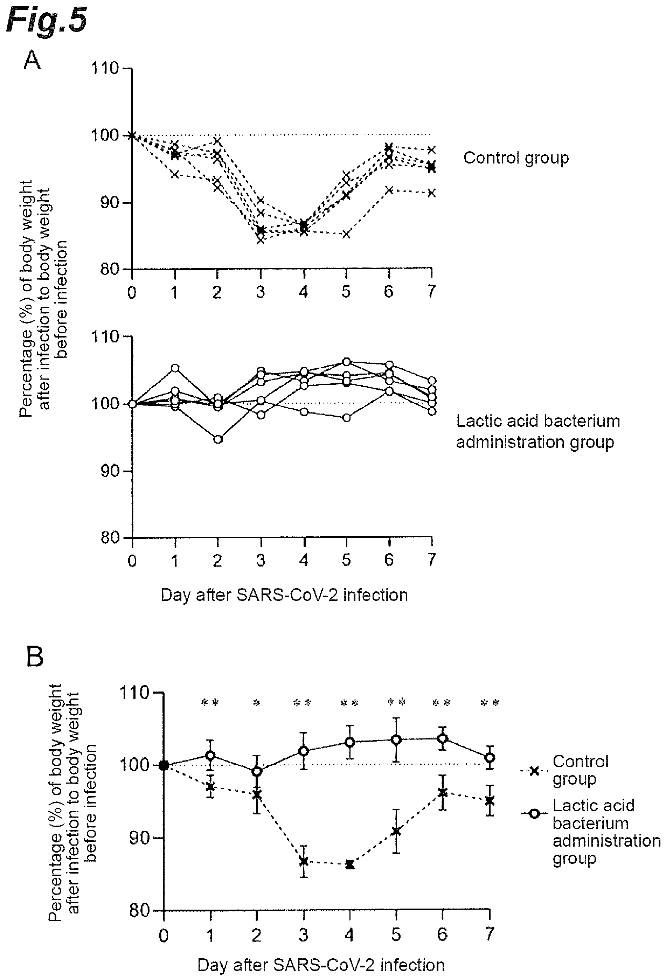

[Fig. 5] Fig. 5A shows the time-course change in the percentage of the post-infection body weight relative to the pre-infection body weight in each mouse of the control group or the lactic acid bacterium administration group in the case of intranasal administration. Fig. 5B shows the time-course change in the average percentage of the post-infection body weight relative to the pre-infection body weight of the control group or the lactic acid bacterium administration group in the case of intranasal administration. Note that in the graph, * indicates p < 0.05, ** indicates p < 0.01 (Mann-Whitney U test), and error bars each indicate the standard deviation.

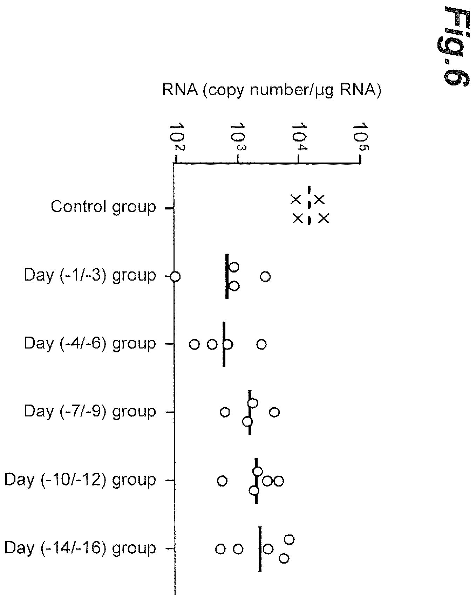

[Fig. 6] Fig. 6 shows the level of SARS-CoV-2 RNA in the NALT of the control or the lactic acid bacterium administration groups (5 groups) at 2 days after infection in the case of intranasal administration. Note that - in the graph indicates the geometric mean.

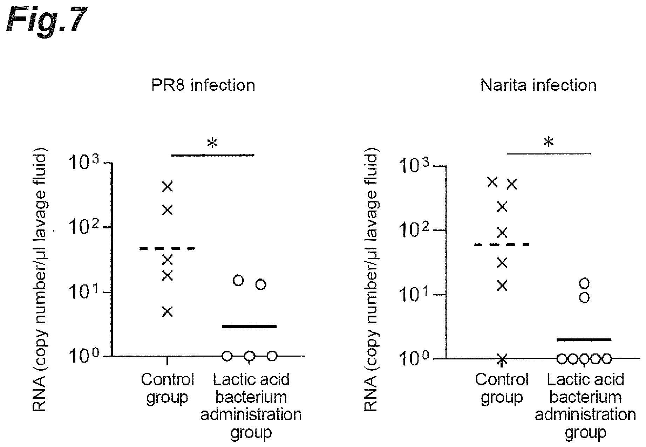

[Fig. 7] Fig. 7 shows the level of influenza A virus (H1N1) RNA in the nasal lavage fluid of the control or the lactic acid bacterium administration group at 2 days after infection in the case of intranasal administration. Note that in the graph, * indicates p < 0.05 (Mann-Whitney U test), and - indicates the geometric mean.

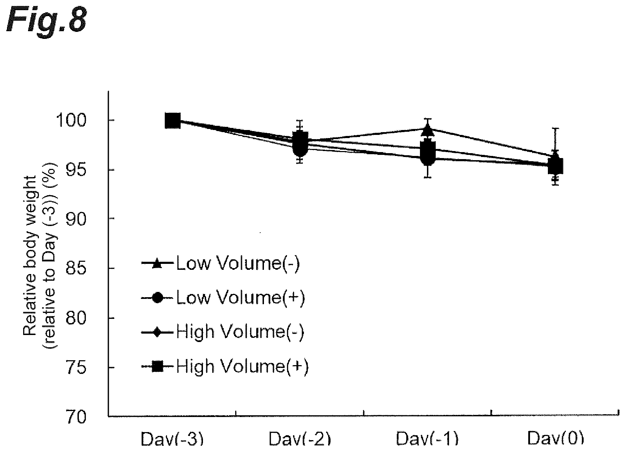

[Fig. 8] Fig. 8 shows the percentage (relative weight (Day (-3) was used as a reference)) (%) of Day (-2), Day (-1), or Day (0) body weight relative to Day (-3) body weight in the Low Volume (-), Low Volume (+), High Volume (-), and High Volume (+) groups. Note that the error bars in the graph each indicate the standard deviation.

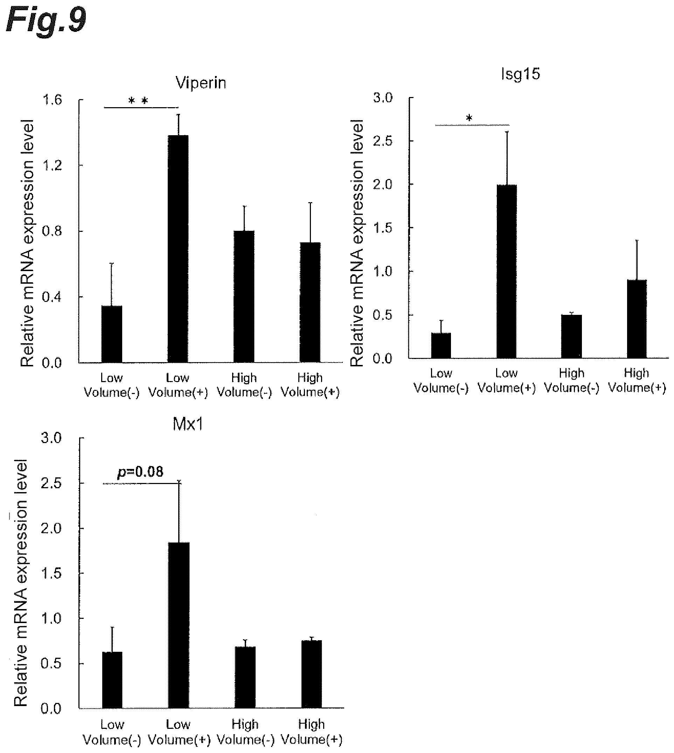

[Fig. 9] Fig. 9 shows the relative mRNA expression level of Viperin, Isg15, or Mx1 in the submandibular lymph nodes of the Low Volume (-), Low Volume (+), High Volume (-), and High Volume (+) groups. Note that * in the graph indicates p < 0.05 and ** indicates p < 0.01 (Student's t-test), and error bars each indicate the standard deviation.

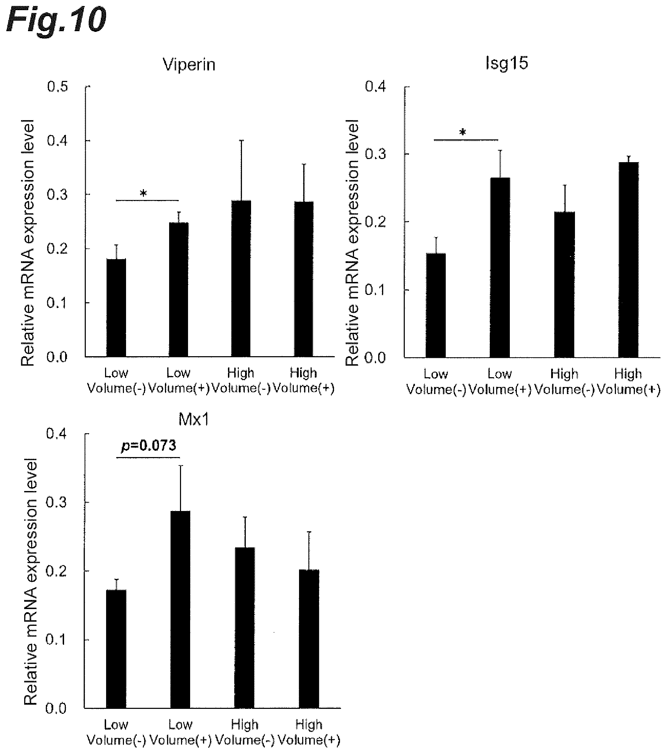

[Fig. 10] Fig. 10 shows the relative mRNA expression level of Viperin, Isg15, or Mx1 in the spleen of the Low Volume (-), Low Volume (+), High Volume (-), and High Volume (+) groups. Note that * in the graphs indicates p < 0.05 (Student's t-test), and error bars each indicate the standard deviation.

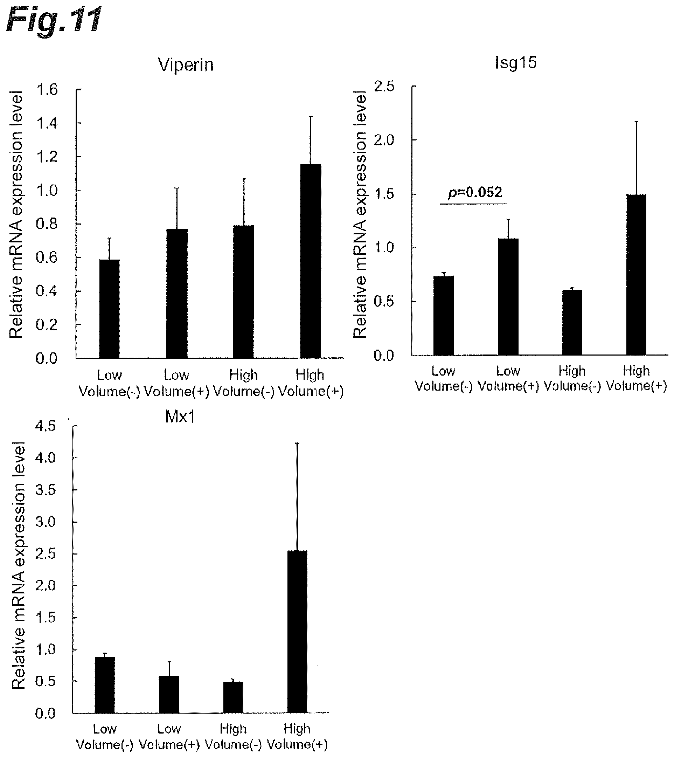

[Fig. 11] Fig. 11 shows the relative mRNA expression level of Viperin, Isg15, or Mx1 in the lung of the Low Volume (-), Low Volume (+), High Volume (-), and High Volume (+) groups. Note that the error bars in the graphs each indicate the standard deviation.

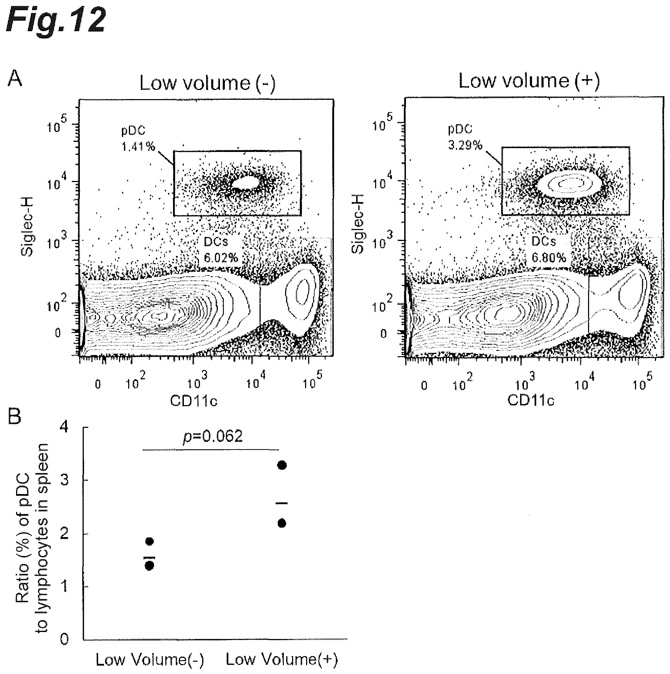

[Fig. 12] Fig. 12A shows FACS analysis images for CD11c and Siglec-H in the lymphocyte fraction of the spleen from the Low Volume (-) or Low Volume (+) group. Note that the CD11c⁺SIglec-H⁺ cell population was analyzed as pDC. Fig. 12B shows the ratio (%) of pDC to lymphocytes in the spleen from the Low Volume (-) or Low Volume (+) group. Note that - in the graph indicates the mean value.

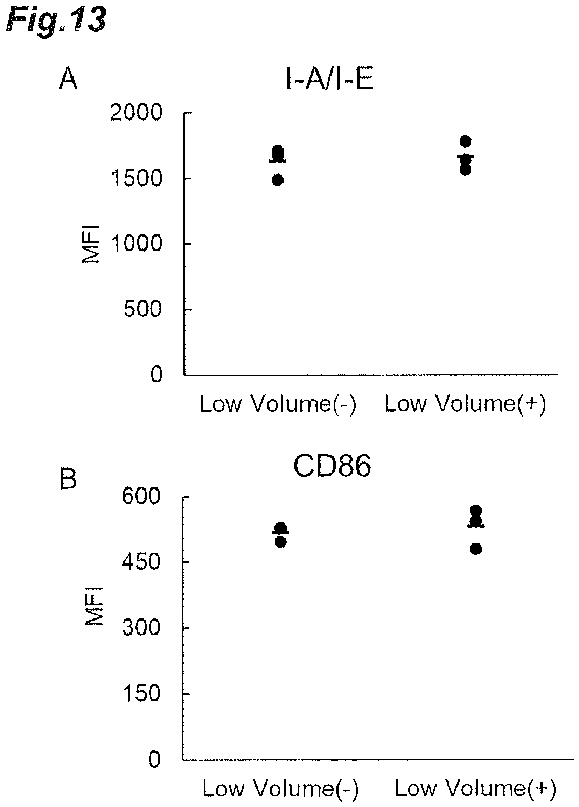

[Fig. 13] Fig. 13 shows the expression levels (MFI: Median Fluorescence Intensity) of MHC class II (I-A/I-E) (A) and CD86 (B) on pDC in the spleen from the Low Volume (-) or Low Volume (+) group. Note that - in the graph indicates the mean value.

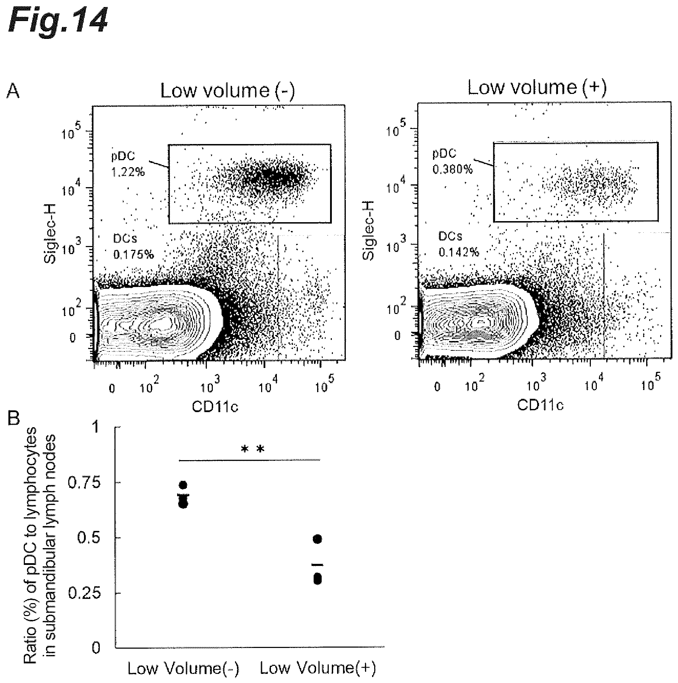

[Fig. 14] Fig. 14A shows FACS analysis images for CD11c and Siglec-H in the lymphocyte fraction of the submandibular lymph nodes from the Low Volume (-) or Low Volume (+) group. Note that the CD11c⁺SIglec-H⁺ cell population was analyzed as pDC. Fig. 12B shows the ratio (%) of pDC to lymphocytes in the submandibular lymph nodes from the Low Volume (-) or Low Volume (+) group. Note that ** in the graph indicates p < 0.01 (Student's t-test), and - indicates the mean value.

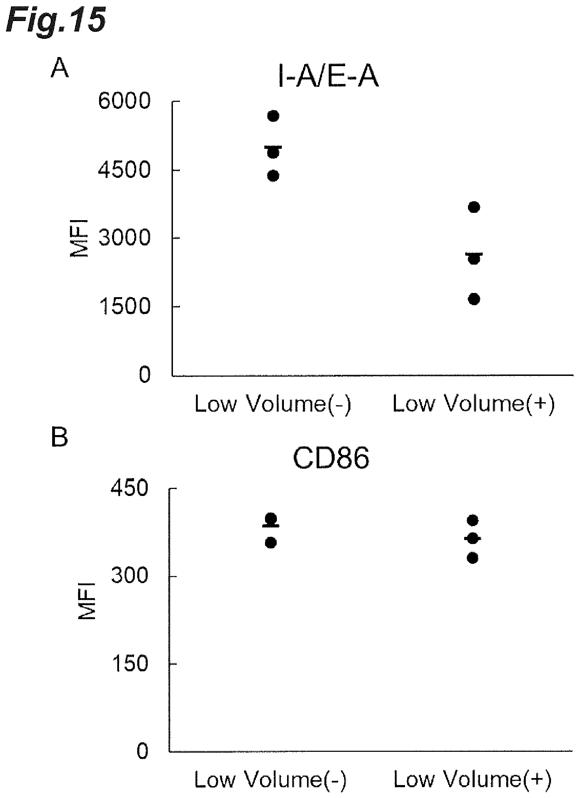

[Fig. 15] Fig. 15 shows the expression levels (MFI) of MHC class II (I-A/I-E) (A) and CD86 (B) on pDC of the submandibular lymph nodes from the Low Volume (-) or Low Volume (+) group. Note that - in the graphs indicates the mean value.

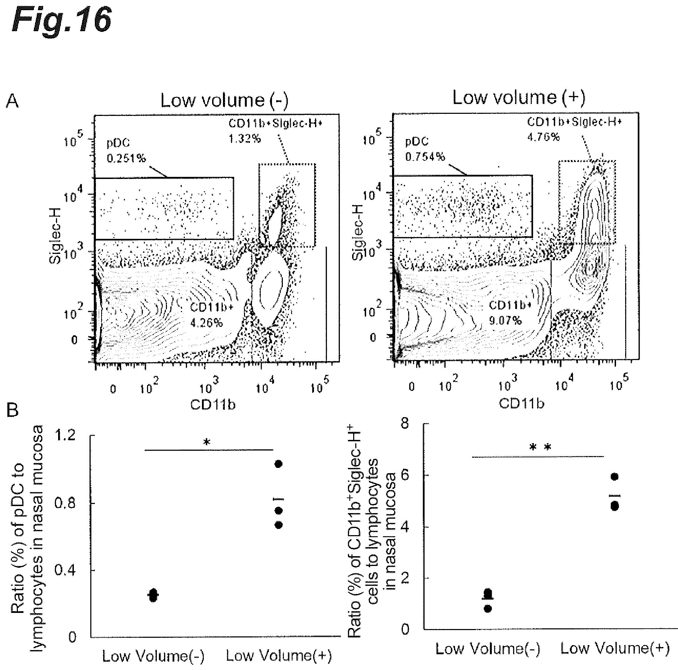

[Fig. 16] Fig. 16A shows FACS analysis images for CD11c and Siglec-H in the lymphocyte fraction of the nasal mucosa from the Low Volume (-) or Low Volume (+) group. The CD11b⁻Siglec-H⁺ cell population was analyzed as pDC and the CD11b⁺Siglec-H⁺ cell population as CD11b⁺Siglec-H⁺ cells. Fig. 16B shows the ratio (%) of pDC or CD11b⁺Siglec-H⁺ cells to lymphocytes in the nasal mucosa from the Low Volume (-) or Low Volume (+) group. Note that in the graphs, * indicates p < 0.05 and ** indicates p < 0.01 (Student's t-test), and - indicates the mean value.

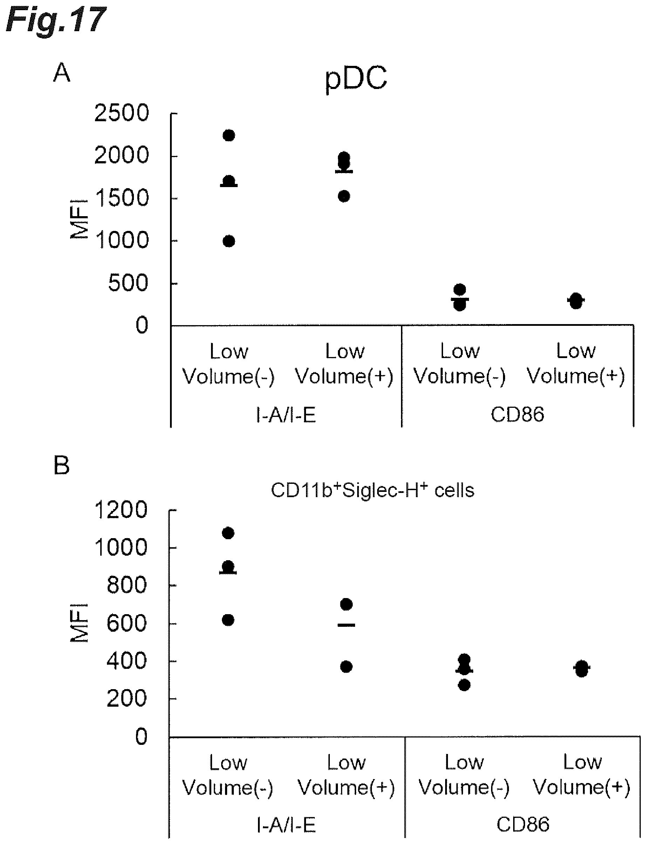

[Fig. 17] Fig. 17A shows the expression levels (MFI) of MHC class II (I-A/I-E) (A) and CD86 (B) on pDC in the nasal mucosa from the Low Volume (-) or Low Volume (+) group. Fig. 17B shows the expression levels (MFI) of MHC class II (I-A/I-E) (A) and CD86 (B) on CD11b⁺Siglec-H⁺ cells in the nasal mucosa from the Low Volume (-) or Low Volume (+) group. Note that - in the graphs indicates the mean value.

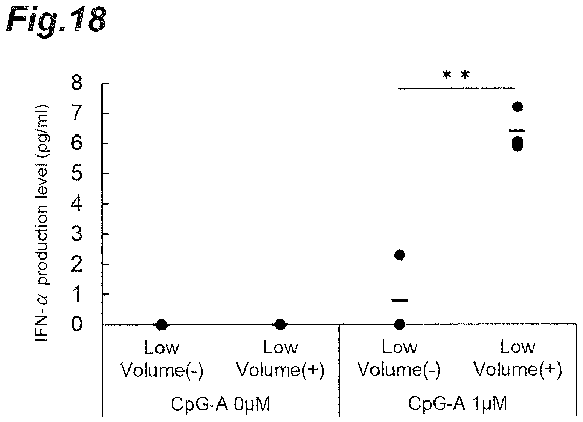

[Fig. 18] Fig. 18 shows the level (pg/ml) of IFN-α produced in the culture supernatant of the culture of nasal mucosa cells collected from the Low Volume (-) or Low Volume (+) group without CpG-A addition (CpG-A 0 µM) and with CpG-A addition (CpG-A 1 µM). Note that ** in the graph indicates p < 0.01 (Student's t-test), and - indicates the mean value.

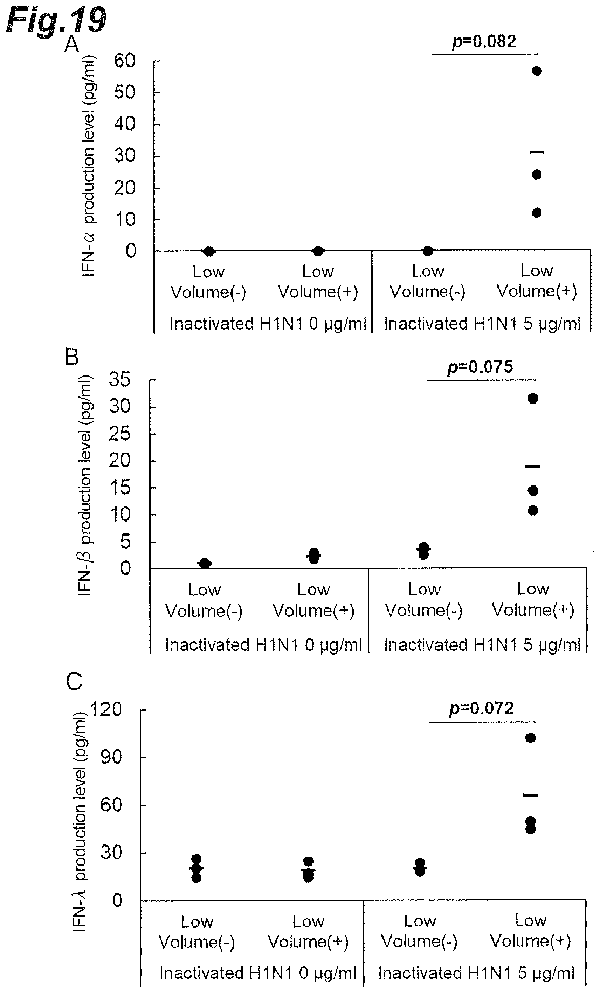

[Fig. 19] Fig. 19 shows the level (pg/ml) of IFN-α produced, the level (pg/ml) of IFN-β produced, or the level (pg/ml) of IFN-λ produced in the culture supernatant of the culture of nasal mucosa cells collected from the Low Volume (-) or Low Volume (+) group without inactivated HIN1 addition (HIN1 0 µg/ml) or with inactivated HIN1 addition (HIN1 5 µg/ml). Note that - in the graphs indicates the mean value.

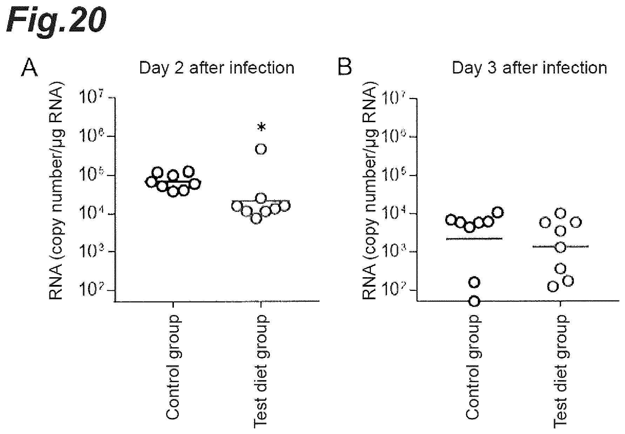

[Fig. 20] Fig. 20A shows the level of SARS-CoV-2 RNA in the NALT of the control group or the lactic acid bacterium administration groups (6 groups) at 2 days after infection in the case of oral administration (* indicates p = 0.0104 (Mann-Whitney test)). Fig. 20B shows the level of SARS-CoV-2 RNA in the NALT of the control or the lactic acid bacterium administration groups (6 groups) at 3 days after infection in the case of oral administration.

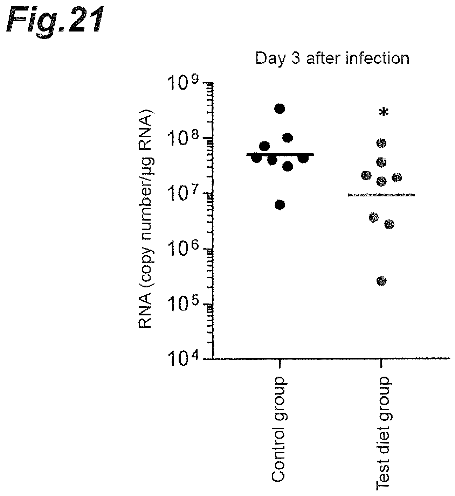

[Fig. 21] Fig. 21 shows the level of SARS-CoV-2 RNA in the lung of the control group or the lactic acid bacterium administration groups (6 groups) at 3 days after infection in the case of oral administration (* indicates p = 0.0281 (Mann-Whitney test)).

### Description of Embodiments

The present invention provides a pharmaceutical composition for topical administration, the composition comprising a lactic acid bacterium as an active ingredient.

The lactic acid bacterium that is an active ingredient in the present invention is not particularly limited, but is preferably a lactic acid bacterium that has a capacity to induce interferon (IFN) production, and is more preferably a lactic acid bacterium that has a capacity to activate plasmacytoid dendritic cells (pDC) and to induce at least IFN production. For example, a lactic acid bacterium may be used that has a capacity to induce production of IFN-α in an amount of 50 pg/mL or more, preferably 100 pg/ml or more, when added at 1.0 µg/ml to a culture medium for plasmacytoid dendritic cells (pDC) derived from human peripheral blood mononuclear cells.

The lactic acid bacterium that is an active ingredient in the present invention may also be a lactic acid bacterium that has a capacity to induce IFN production in a subject after the subject of administration of the pharmaceutical composition of the present invention contracts an infectious disease.

Preferably, the lactic acid bacterium having a capacity to induce IFN production in the present invention can induce production of any of one or more IFNs of Type I IFN (Type I interferon), Type II IFN (Type II interferon), and Type III IFN (Type III interferon). Type I IFNs are cytokines that are considered effective against viral infection and include IFN-α (1, 2, 4, 5, 6, 7, 8, 10, 13, 14, 16, 17, 21) and IFN-β. Type II IFNs include IFN-γ and Type III IFNs include IFN-λ. Among these lactic acid bacteria having a capacity to induce IFN production in the present invention, those having activity to induce production of Type I IFN are especially preferred. When pDCs are activated by a lactic acid bacterium having a capacity to induce IFN production in the present invention, cellular protrusions characteristic of activated pDC appear and the production of Type I IFN and Type III IFN is induced, and in addition, the production of Type II IFN such as IFN-γ from NK cells or Th1 cells can also be induced.

It is preferable that the IFN, the production of which can be induced by the lactic acid bacterium that has a capacity to induce IFN production and is an active ingredient in the present invention, is not particularly limited, but is one or more kinds selected from the group consisting of IFN-α, IFN-β and IFN-λ, and it is more preferable that the IFN is two or more kinds selected from the group consisting of IFN-α, IFN-β and IFN-λ, and it is still more preferable that the IFN is two or more kinds selected from the group consisting of IFN-α, IFN-β and IFN-λ while at least one kind of the two or more kinds of IFNs is IFN-α, and it is still much more preferable that at least two kinds of the two or more IFNs are IFN-α and IFN-β, and it is particularly preferable that the INF is three kinds including IFN-α, IFN-β and IFN-λ.

Whether the lactic acid bacterium that has a capacity to induce IFN production and is an active ingredient in the present invention can activate pDCs and can induce at least IFN production may be checked, for example, by measuring whether pDCs are activated and IFN production is induced when the candidate lactic acid bacterium is co-cultured in the presence of mammalian (e.g., mouse) bone marrow cells. For example, IFN-α production can be measured, for instance, by ELISA for IFN-α concentration in the culture medium, specifically by suspending erythrocyte-depleted mouse bone marrow cells at 5 × 10⁵ cells /ml in RPMI medium (Sigma) containing 10% fetal calf serum (FCS) and 2 µM of β-mercaptoethanol, adding, to the resulting cell suspension, Flt-3L as a pDC-inducing cytokine at a final concentration of 100 ng/ml, culturing the cells in a CO₂ incubator at 37°C and 5% CO₂, adding, after 7 days, a lactic acid bacterium strain at 10 µg/ml, collecting the culture supernatant after 48 hours, and measuring the IFN-α concentration in the culture supernatant by ELISA using an IFN-α assay kit (PBL, Inc.).

The lactic acid bacterium that is an active ingredient in the present invention is not particularly limited and include lactic acid bacteria, and examples thereof include lactic acid bacteria belonging to the genus *Lactococcus,* the genus *Leuconostoc,* the genus *Pediococcus,* the genus *Streptococcus,* the genus *Enterococcus,* the genus *Lactobacillus* and the genus *Oenococcus,* the genus *Bifidobacterium,* the genus *Weissella,* and the genus *Tetragenococcus.* The lactic acid bacterium that is an active ingredient in the present invention is preferably a lactic acid bacterium belonging to the genus *Lactococcus* or the genus *Pediococcus.* One species or strain of bacterium, namely the lactic acid bacterium that is an active ingredient in the present invention, may be used, or two or more species or strains thereof may also be used in combination.

Examples of the *Lactococcus* bacterium include *Lactococcus lactis, Lactococcus lactis* subsp. *lactis, Lactococcus garvieae, Lactococcus lactis* subsp. *cremoris,* or *Lactococcus lactis* subsp. *hordniae,* and *Lactococcus lactis* subsp. *lactis* is preferred from the viewpoint of its ability to activate pDC and induce at least IFN production.

Specific examples of the *Lactococcus* bacterium include, *Lactococcus lactis* subsp. *lactis* JCM5805, *Lactococcus lactis* subsp. *lactis* NBRC12007, *Lactococcus lactis* subsp. *lactis* NRIC1150, *Lactococcus lactis* subsp. *lactis* JCM20101, *Lactococcus lactis* subsp. *lactis* JCM7638, *Lactococcus lactis* subsp. *lactis* ATCC11454, *Lactococcus garvieae* NBRC100934, *Lactococcus lactis* subsp. *cremoris* JCM16167, *Lactococcus lactis* subsp. *cremoris* NBRC100676, *Lactococcus lactis* subsp. *hordniae* JCM1180, *Lactococcus lactis* subsp. *hordniae* JCM11040, or *Lactococcus plantarum* JCM11056, and *Lactococcus lactis* subsp. *lactis* JCM5805 is preferred from the viewpoint of its ability to activate pDC and induce at least IFN production.

Examples of the *Leuconostoc* bacterium include *Leuconostoc lactis.* Specific examples of the *Leuconostoc* bacterium include *Leuconostoc lactis* NBRC12455.

Examples of the *Pediococcus* bacterium include *Pediococcus acidilactici, Pediococcus pentosaceus, Pediococcus cellicola, Pediococcus claussenii, Pediococcus damnosus, Pediococcus ethanolidurans, Pediococcus inopinatus, Pediococcus parvulus,* or *Pediococcus stilesii.* Specific examples of the *Pediococcus* bacterium include *Pediococcus acidilactici* JCM8797, *Pediococcus acidilactici* K15, or *Pediococcus damnosus* JCM5886.

Examples of the *Streptococcus* bacterium include *Streptococcus thermophilus.* Specific examples of the *Streptococcus* bacterium include *Streptococcus thermophilus* SBC 8781.

Examples of the *Enterococcus* bacterium include *Enterococcus alcedinis.*

Examples of the *Lactobacillus* bacterium include *Lactobacillus paracasei, Lactobacillus delbrueckii, Lactobacillus acidophilus, Lactobacillus casei, Lactobacillus fructivorans, Lactobacillus hilgardii, Lactobacillus rhamnosus, Lactobacillus plantarum, Lactobacillus gasseri, Lactobacillus acidophilus,* or *Lactobacillus bulgaricus.* Specific examples of the *Lactobacillus* bacterium include *Lactobacillus paracasei* KW3110, *Lactobacillus paracasei* MCC1849, *Lactobacillus paracasei* K71, *Lactobacillus rhamnosus* GG, *Lactobacillus rhamnosus* CRL1505, *Lactobacillus gasseri* SBT2055, *Lactobacillus parakefiri* (*Lentilactobacillus parakefiri* in the new classification) JCM8573, *Lactobacillus pentosus* (*Lactiplantibacillus pentosus* in the new classification) ONRICb0240, *Lactobacillus plantarum* (*Lactiplantibacillus plantarum* in the new classification) L-137, or *Lactobacillus acidophilus* L-92 or *Lactobacillus bulgaricus* OLL1073R-1 (including the corresponding strain with extracellular polysaccharides). In the present invention, from the viewpoint of excellent water dispersibility in beverages, the *Lactobacillus* bacterium may be, for example, one or two or more species selected from the group consisting of *Lactobacillus paracasei, Lactobacillus delbrueckii, Lactobacillus acidophilus, Lactobacillus casei, Lactobacillus fructivorans, Lactobacillus hilgardii,* and *Lactobacillus rhamnosus.*

Examples of the *Oenococcus* bacterium include *Oenococcus oeni.* Specific examples of the *Oenococcus* bacterium include *Oenococcus oeni* JCM6125.

Examples of the *Bifidobacterium* bacterium include *Bifidobacterium animalis* subsp. *lactis* or *Bifidobacterium longum* subsp. *infantis.* Specific examples of the *Bifidobacterium* bacterium include *Bifidobacterium animalis* subsp. *lactis* JCM10602 or *Bifidobacterium longum* subsp. *infantis* JCM1222.

Examples of the *Weissella* bacterium include *Weissella paramesenteroides* or *Weissella viridescens.* Specific examples of the *Weissella* bacterium include *Weissella paramesenteroides* JCM9890 or *Weissella viridescens* JCM1174.

Examples of the *Tetragenococcus* bacterium include *Tetragenococcus halophilus.* Specific examples of the *Tetragenococcus* bacterium include *Tetragenococcus halophilus* NRIC0098.

In the present invention, lactic acid bacteria are available from known depository institutions or other sources; for example, the JCM strains among the above *Lactococcus* strains are available from the Microbial Materials Development Laboratory, RIKEN BioResource Center (3-1-1, Koyadai, Tsukuba, Ibaraki); the NBRC strains are available from Biological Resource Center, the National Institute of Technology and Evaluation (2-5-8, Kazusakamatari, Kisarazu, Chiba); the NRIC strains are available from Tokyo University of Agriculture, Culture Collection Center(1-1-1 Sakuragaoka, Setagaya-ku, Tokyo); and the ATCC strains are available from American Type Culture Collection (USA), respectively.

The lactic acid bacterium used as an active ingredient in the present invention may include a culture of lactic acid bacterium. Examples of the culture include live bacteria, dead bacteria, crushed live or dead bacteria, lyophilized live or dead bacteria, crushed lyophilized live or dead bacteria, enzyme-treated live or dead bacteria, culture media, culture medium extracts as well as parts of lactic acid bacterium and processed lactic acid bacterium products. The dead bacteria may be obtained by treating live bacteria, and may be obtained, for example, by a combination of any one or two or more of heat treatment, treatment with drugs (e.g., antibiotics), treatment with chemicals (e.g., formalin), UV treatment, and treatment with radiation (e.g., γ-rays). Examples of the processed products include heated bacterial cells (dead bacteria), lyophilized products thereof, and cultures containing these materials, as well as liquids obtained by crushing bacterial cells by, for instance, sonication and enzyme-treated bacterial cell liquids. In addition, examples of the processed products also include processed products in which cell walls are removed by enzymatic or mechanical means. Further included is a nucleic acid-containing fraction obtained by dissolving bacterial cells with, for instance, a surfactant and then precipitating them with, for instance, ethanol. Furthermore, the lactic acid bacterium used as an active ingredient in the present invention may also include a dead bacterium. Moreover, the above lactic acid bacterium culture may contain DNA and RNA derived from the lactic acid bacterium, and the DNA and RNA derived from the lactic acid bacterium can preferably activate pDC and induce at least IFN production.

The lactic acid bacterium may be cultured by known procedures using a known culture medium. Examples of the culture medium that can be used include MRS medium, GAM medium, or LM17 medium, and inorganic salts, vitamins, amino acids, antibiotics, and serum may be added as needed and then used. The culturing should be performed at 25 to 40°C for a few hours to a few days.

After culturing, the lactic acid bacterium cells may be collected by centrifugation or filtration to obtain the lactic acid bacterium cells. When used as a dead bacterium, the bacterium may be sterilized and inactivated using, for instance, an autoclave, and then used.

In the present invention, the topical administration means local administration to the site where the lactic acid bacterium, an active ingredient, acts directly, and examples include intranasal, sublingual, inhalation, buccal, transdermal, transrectal, transvaginal, transpulmonary, transairway (intratracheal), or ophthalmic administration. The topical administration is preferably intranasal, sublingual, or inhalation administration in order to increase the efficacy of infection prevention or infection suppression and to reduce the burden of administration. In the topical administration, it is assumed that the lactic acid bacterium, an active ingredient, is absorbed through the mucosa at local sites such as the nasal cavity, sublingual, pharynx, larynx, respiratory tract, bronchi, lungs, vagina, skins, eyes, and intestines. When the lactic acid bacterium is administered through the nasal cavity, the site of administration is preferably the Waldeyer's pharyngeal ring (e.g., pharyngeal tonsil, upper pharynx (nasopharynx), palatine tonsil, oropharynx or lingual tonsil). The topical administration is advantageous in that, compared to oral administration, namely administration as an oral drug, etc., the lactic acid bacterium, an active ingredient, that has a capacity to activate pDC and induce at least IFN production can act directly on pDC and conventional dendritic cells (cDC) present around the local sites, thereby enhancing the efficacy of infection prevention or infection suppression.

The dosage forms (preparations) suitable for topical administration in the present invention are well known to those skilled in the art and may be selected, if appropriate, depending on the purpose. Examples of the dosage forms include nasal drops (e.g., nasal powder, nasal liquid), inhalants (e.g., inhalant aerosol, inhalant powder, inhalant liquid), sublingual agents, buccal administration agents, injections (including intradermal, subcutaneous, intramuscular, or intravenous injections), ointments, creams, gels, suppositories, patches, and poultices. These dosage forms may be formulated (as preparations) using pharmaceutically acceptable carriers (including solvents) according to methods commonly practiced in the art (e.g., known methods described in the General Pharmaceutical Preparation Regulations of the Japanese Pharmacopoeia, 18th Revision, etc.).

In the present invention, the nasal drops are not particularly limited, but may be prepared, for example, by dispersing a lactic acid bacterium in a liquid (e.g., purified water or saline) or in powder made of a carrier, or the like. The lactic acid bacterium can also be provided as nasal drops by filling into a known atomizer for nasal drops, etc.

In the present invention, the inhalants are not particularly limited, but may be prepared, for example, by dispersing anlactic acid bacterium in a liquid (e.g., purified water or saline) or in powder made of a carrier, or the like, and may be provided as an inhalant by filling into, for instance, a known inhaler for the inhalant. The present invention may also be provided by means of inhalation of water particles or water vapor together with a lactic acid bacterium, for instance, orally or intranasally. Specific examples of the means include a method of inhalation with a nebulizer or other inhaler capable of inhalation of water particles, or a method of inhalation with a steam generator capable of generating water vapor upon generation of heat through a reaction with oxygen in the air and supplying the heated water vapor to a subject.

It is preferable that the nebulizer is an ultrasonic nebulizer, mesh nebulizer, or compressor type nebulizer.

It is preferable that the particle size of the inhaled water particles is less than 10 µm, and it is more preferable that the particle size is from 1 to 8 µm. Here, the particle size of water particles can be measured by light scattering.

In formulation, examples of the pharmaceutically acceptable carriers include, but are not limited to, excipients, viscosity agents, lubricants, binders, disintegrants, solvents, dissolution aids, suspending agents, emulsifiers, isotonic agents, buffers, soothing agents, and stabilizers. In addition, additives may be optionally included, such as preservatives (antiseptics), pH adjusters, coolants, antioxidants, wetting agents, adhesives, and deodorants.

Examples of the excipients include lactose, white sugar, D-mannitol, starch, cornstarch, crystalline cellulose, and light anhydrous silicic acid.

Examples of the viscosity agents include polyhydric alcohols (e.g., glycerin), celluloses (e.g., methylcellulose, carboxymethylcellulose, hydroxypropylmethylcellulose), hydrophilic polymers (e.g., polyvinyl alcohol, polyvinylpyrrolidone, carboxyvinyl polymers, sodium carboxymethylcellulose), sodium alginate, chondroitin sulfate, and polyethylene glycol.

Examples of the lubricants include magnesium stearate, calcium stearate, talc, and colloidal silica.

Examples of the binders include crystalline cellulose, white sugar, dextrin, hydroxypropyl cellulose, hydroxypropyl methyl cellulose, polyvinylpyrrolidone, starch, sucrose, gelatin, methylcellulose, and sodium carboxymethylcellulose.

Examples of the disintegrants include starch, carboxymethylcellulose, calcium carboxymethylcellulose, and L-hydroxypropylcellulose.

Examples of the solvents include water (including purified water, pure water, etc.), ethanol, isopropyl alcohol, and propylene glycol.

Examples of the dissolution aids include celluloses (e.g., methylcellulose, carboxymethylcellulose, hydroxypropylmethylcellulose); and polyethylene glycol, propylene glycol, ethanol, tris-aminomethane, cholesterol, triethanolamine, sodium carbonate, sodium citrate, and polyvinylpyrrolidone.

Examples of the suspending agents include surfactants (e.g., sodium lauryl sulfate, lauryl aminopropionic acid, lecithin, benzalkonium chloride, glycerin monostearate, polyoxyethylene hardened castor oil, polysorbate), polyhydric alcohols (e.g., glycerin), sugars (e.g., sorbitol, mannitol, sucrose), celluloses (e.g., methylcellulose, carboxymethylcellulose, hydroxypropylmethylcellulose), hydrophilic polymers (e.g., polyvinyl alcohol, polyvinylpyrrolidone, carboxyvinyl polymers), and chondroitin sulfate.

Examples of the isotonic agents include dextrose, D-sorbitol, sodium chloride, glycerin, potassium chloride, propylene glycol, and sucrose.

Examples of the buffers include phosphates (e.g., sodium hydrogen phosphate, sodium dihydrogen phosphate), boric acid, borax, acetates (e.g., sodium acetate), carbonates (e.g., sodium carbonate, calcium carbonate, potassium carbonate), citric acid, and sodium L-glutamate.

Examples of the soothing agents include benzyl alcohol, chlorobutanol, propylene glycol, ethyl aminobenzoate, and lidocaine.

Examples of the stabilizers include sulfur compounds (e.g., sodium sulfite, sodium hydrogen sulfite, sodium hydrogen metasulfite, sodium thiosulfate, Rongalite, thioglycerol, thioglycolic acid, thiolactic acid, cysteine, glutathione, thioacetic acid, methionine, thiosorbitol, thioglucose, thiourea), inorganic acids and their salts (e.g., boric acid, borax, phosphoric acid, metaphosphoric acid, sodium carbonate, sodium bicarbonate), organic acids (e.g., formic acid, oxalic acid, tartaric acid, citric acid, edetic acid) and their salts (e.g., sodium edetate), acid amides (e.g., acetamide, diethylacetamide, nicotinic acid amide, urea, barbital), urea derivatives, polyhydric alcohols (e.g., glycol, propylene glycol, glycerin, polyethylene glycol, dextrose, ascorbic acid), sugars, phenols (e.g., phenol, quinone, coumarone, isocoumarone), dibutylhydroxytoluene, and amino acids and proteins (e.g., glycine, glutamic acid, lysine, phenylalanine, casein, edestin).

Examples of the emulsifiers include glycerol esters (glycerol monooleate), sucrose fatty acid esters, lecithins (e.g., plant lecithin, egg yolk lecithin, soy lecithin), various surfactants (alkyl benzene sulfonate emulsifiers, benzalkonium chloride, sorbitan sesquioleate, dodecylbenzene sulfonate), and triethanolamine.

Examples of the preservatives (antiseptics) include paraoxybenzoates (e.g., propyl paraoxybenzoate, butyl paraoxybenzoate), parabens (e.g., methylparaben, ethylparaben, propylparaben, butylparaben), invert soaps (e.g., benzalkonium chloride, benzethonium chloride, chlorhexidine gluconate, cetylpyridium chloride), alcohol derivatives (e.g., benzyl alcohol, phenethyl alcohol), organic acids and their salts (e.g., sodium dehydroacetate, sorbic acid, sodium sorbate), and phenols (e.g., para-chloromethoxyphenol, para-chloromethacresol).

Examples of the pH adjusters include sodium hydroxide, potassium hydroxide, trisodium phosphate, disodium hydrogen phosphate, hydrochloric acid, nitric acid, citric acid, boric acid, and acetic acid.

Examples of the coolants include 1-menthol, camphor, and peppermint water. Examples of the antioxidants include sulfites, ascorbic acid, citric acid, and sodium edetate.

Examples of the wetting agents include propylene glycol, polysorbate, polyethylene glycol, and glycerin.

Examples of the adhesives include hydroxypropyl cellulose, hydroxypropyl methylcellulose, carboxy vinyl polymers, propylene glycol, and polysorbate 80.

Examples of the deodorants include trehalose, malic acid, maltose, anise essential oil, vanilla essential oil, and cardamom essential oil.

The pharmaceutical composition of the present invention may be used for the prevention or treatment of infectious diseases.

The pharmaceutical composition of the present invention may be used to reduce the risk of contracting an infectious disease in a subject of administration or to reduce the risk of infection with a pathogen in a subject of administration. The subject of administration is a subject to whom the pharmaceutical composition is administered and may be a subject at risk of contracting an infectious disease or a subject at risk of infection with a pathogen. In the present invention, the wording "to reduce the risk of contracting an infectious disease" means that the probability of contracting an infectious disease is reduced, and the wording "to reduce the risk of infection with a pathogen" means that the probability of infection with a pathogen is reduced.

Also, one embodiment of the present invention may be a composition for topical administration containing *Lactococcus lactis* subsp. *lactis* as an active ingredient, and the composition is preferably a pharmaceutical composition.

In the present invention, the infectious disease is not particularly limited and is a disease caused by infection with a pathogen, and examples thereof include viral infections, bacterial infections, rickettsia·chlamydia infections, fungal infections, parasitic infections, and prion infections, and the infectious disease is preferably viral and bacterial infections.

In the present invention, examples of the viral infections include COVID-19, influenza virus infection, rabies, Japanese encephalitis, West Nile fever, dengue fever, chikungunya fever, Zika virus infection, tick-borne encephalitis, hepatitis E, severe febrile thrombocytopenia syndrome (SFTS), Ebola hemorrhagic fever, severe acute respiratory syndrome (SARS), and Middle East respiratory syndrome (MERS).

In the present invention, examples of the bacterial infections include Q fever, plague, salmonellosis, leptospirosis, cat scratch disease, brucellosis, *Capnocytophaga canimorsus* infection, *Corynebacterium ulcerans* infection, campylobacteriosis, and anthrax.

In the present invention, examples of the rickettsia·chlamydia infections include Japanese spotted fever, tsutsugamushi disease, and psittacosis.

In the present invention, examples of the fungal infections include dermatophytosis and cryptococcosis.

In the present invention, examples of the parasitic infections include toxoplasmosis, ascariasis, echinococcosis, cryptosporidiosis, and anisakiasis.

In the present invention, examples of the prion infections include variant Creutzfeldt-Jakob disease (vCJD).

In the present invention, the wording "prevention of a viral infectious disease" used means to include prevention of the onset of a viral infectious disease and prevention of the aggravation of the viral infectious disease. Here, the presence or absence of the onset of a viral infectious disease may be determined, for example, by known methods such as PCR or antigen and antibody tests. The presence or absence of the aggravation of the viral infectious disease may be determined using, for instance, the presence or absence of respiratory failure, the oxygen saturation level, and pneumonia findings as an indicator, and if the viral infectious disease is COVID-19, it is possible to consult the severity classification, etc., as described in the U.S. National Institute of Health (NIH) treatment guidelines for COVID-19 (https://www.covid19treatmentguidelines.nih.gov/tables/management-of-hospitalized-adults-summary/).

Examples of the viruses causing viral infectious diseases that are prevented or treated using the pharmaceutical composition of the present invention include, but are not particularly limited to, SARS-CoV-2, severe acute respiratory syndrome (SARS) coronavirus, Middle East respiratory syndrome coronavirus, conventional human coronaviruses (229E, NL63, OC43, and HKU1), influenza virus, parainfluenza virus, adenovirus, RS virus, human metapneumovirus, rhinovirus, dengue virus, varicella-zoster virus, herpes simplex virus, measles virus, parainfluenza virus, enterovirus, rhinovirus, human metapneumovirus, alpha virus, cytomegalovirus, Epstein-Barr virus, yellow fever virus, echovirus, coxsackie virus, herpes simplex virus, measles virus, rubella virus, varicella virus, arbovirus, arenavirus, filovirus, and human papillomavirus. In the present invention, the viruses causing viral infectious diseases are preferably viruses causing respiratory infections, and more preferably SARS-CoV-2.

In the present invention, SARS-CoV-2 includes not only the first viral strain discovered, but also its variants (e.g., B.1.1.7 strain (alpha variant), B.1.351 strain (beta variant), P.1 strain (gamma variant), B.1.617.2 strain (delta variant), and B.1.1.529 strain (omicron variant)). Note that SARS-CoV-2 is synonymous with severe acute respiratory syndrome coronavirus-2, and SARS-CoV-2 infections (diseases and symptoms caused by SARS-CoV-2 infection) are called COVID-19 (so-called novel coronavirus infection).

The lactic acid bacterium that has a capacity to induce IFN production and is an active ingredient in the present invention can induce IFN production *in vivo* and IFN is not directly administered to a living body. The case where IFN is administered directly to a living body may involve oral administration or parenteral administration. Here, when IFN is directly administered orally to a living body, the IFN is less resistant to gastric and/or intestinal juice and degraded, so that it is unlikely that the IFN is absorbed as it is by the living body. In addition, when IFN is directly administered parenterally to a living body, for example, subcutaneous administration may be allowed, but the intake burden is high and the possibility of side effects (e.g., fever, headache, depression) has been reported. Therefore, the lactic acid bacterium that has a capacity to induce IFN production and is an active ingredient in the present invention is superior to the case of direct administration of IFN to a living body in terms of reduced administration burden, reduced possibility of side effects, and preventive and therapeutic effects against infectious diseases.

As for the mechanism of viral infection, in the case of SARS-CoV-2, for example, it is known that the virus binds to and is adsorbed onto ACE2 (angiotensin converting enzyme 2) receptor on the cell surface, which serves as a foothold for the virus to enter the cell, leading to infection. Thus, the topical administration of lactic acid bacterium, an active ingredient in the present invention, to tissues where ACE2 receptor is highly expressed can exert higher prophylactic and therapeutic effects on infectious diseases. The tissues where ACE2 receptor is highly expressed include the respiratory members (e.g., the nasal cavity, pharynx, larynx, trachea, bronchi, lungs), preferably the upper respiratory tract (more preferably the nasal cavity) and the lower respiratory tract.

The Examples below have demonstrated that the lactic acid bacterium, an active ingredient in the present invention, may be topically administered to elicit an immunostimulatory effect. Thus, according to another aspect of the invention, there is provided an immunostimulating composition for topical administration which contains a lactic acid bacterium as an active ingredient. The immunostimulating composition of the present invention may be a pharmaceutical composition. Here, immunostimulation in the present invention means maintaining or enhancing immune functions, maintaining or enhancing immunity, maintaining or enhancing immune action, or maintaining or enhancing immune response. The effect of immunostimulation may be checked, for example, by an increase in the ratio of pDC to lymphocytes, as shown in the Examples below.

As shown in the Examples below, the lactic acid bacterium, an active ingredient in the present invention, can have a capacity to enhance the expression of IFN-inducible antiviral genes (ISG: IFN-stimulated genes) in the submandibular lymph nodes and/or spleen. In other words, the pharmaceutical composition of the present invention may be used to enhance the expression of ISG in the submandibular lymph nodes and/or spleen. Examples of the ISG, the expression of which can be enhanced by the lactic acid bacterium, an active ingredient in the present invention, include, but are not limited to, Viperin, Isg15, Mx1, Oasl2, IFITM, ISG20, and RyDEN. As for the pharmaceutical composition of the present invention, the ISGs are one or two or more kinds selected from the group consisting of Viperin, Isg15, and Mx1, preferably one or two or more kinds selected from the group consisting of Viperin, Isg15, and Mx1, and more preferably Viperin or ISG15. Here, the Viperin protein, encoded by Viperin, is a radical SAM domain-containing molecule with diverse antiviral activity; ISG15, encoded by *isg15,* is a ubiquitin-like small molecule with antiviral properties; MX1, encoded by *mxl,* is a dynamin-like GTPase, and is known to target the nucleocapsid of the virus and suppress it before replication is established (Schoggins JW., Curr Opin Virol., 2014: 40-46.).

The Examples below have demonstrated that the lactic acid bacterium, an active ingredient in the present invention, can increase the ratio of pDC to lymphocytes in the spleen and the ratio of pDC to lymphocytes in the nasal mucosa. That is, the pharmaceutical composition of the present invention may be used to increase the ratio of pDC to lymphocytes in the spleen and/or the ratio of pDC to lymphocytes in the nasal mucosa. In the present invention, the ratio of pDC to lymphocytes in the spleen herein means the ratio of pDC to all lymphocytes contained in the spleen, and the ratio of pDC to lymphocytes in the nasal mucosa means the ratio of pDC to all lymphocytes contained in the nasal mucosa. The ratio of pDC to lymphocytes in the spleen and/or the ratio of pDC to lymphocytes in the nasal mucosa is not limited, but can be calculated, for example, as the ratio (percentage) of pDC to all lymphocytes in the lymphocyte fraction in analysis using FACS (fluorescence activated cell sorter) as shown in, for example, the Examples below.

In the present invention, the wording "increase the ratio of pDC to lymphocytes in the spleen" means an increase in the ratio of pDC to lymphocytes in the spleen when the lactic acid bacterium is administered, compared to the corresponding ratio when the lactic acid bacterium is not administered. Specifically, when the ratio of pDC to lymphocytes in the spleen of a subject after administration of the lactic acid bacterium is, for example, 1.1-fold, 1.2-fold, 1.3-fold, 1.4-fold, 1.5-fold, 1.6-fold, 1.7-fold, 1.8-fold, 1.9-fold, 2.0-fold, 2.1-fold, 2.2-fold, 2.3-fold, 2.4-fold, 2.5-fold, 2.6-fold, 2.7-fold, 2.8-fold, 2.9-fold, or 3.0-fold or more higher than the corresponding ratio in the subject before administration to the subject, the ratio of pDC to lymphocytes in the spleen can be said to have increased.

In the present invention, the wording "increase the ratio of pDC to lymphocytes in the nasal mucosa" means an increase in the ratio of pDC to lymphocytes in the nasal mucosa when the lactic acid bacterium is administered, compared to the corresponding ratio when the lactic acid bacterium is not administered. Specifically, when the ratio of pDC to lymphocytes in the nasal mucosa of a subject after administration of the lactic acid bacterium is, for example, 1.1-fold, 1.2-fold, 1.3-fold, 1.4-fold, 1.5-fold, 1.6-fold, 1.7-fold, 1.8-fold, 1.9-fold, 2.0-fold, 2.1-fold, 2.2-fold, 2.3-fold, 2.4-fold, 2.5-fold, 2.6-fold, 2.7-fold, 2.8-fold, 2.9-fold, 3.0-fold, 3.1-fold, 3.2-fold, 3.3-fold, 3.4-fold, 3.5-fold, 3.6-fold, 3.7-fold, 3.8-fold, 3.9-fold, 4.0-fold, 4.1-fold, 4.2-fold, 4.3-fold, 4.4-fold, 4.5-fold, 4.6-fold, 4.7-fold, 4.8-fold, 4.9-fold, or 5.0-fold or more higher than the corresponding ratio in the subject before administration to the subject, the ratio of pDC to lymphocytes in the nasal mucosa can be said to have increased.

The Examples below have demonstrated that the lactic acid bacterium, an active ingredient in the present invention, can increase the ratio of CD11b⁺Siglec-H⁺ cells to lymphocytes in the nasal mucosa. That is, the immunostimulating composition of the present invention may be used to increase the ratio of CD11b⁺Siglec-H⁺ cells to lymphocytes in the nasal mucosa. In the present invention, the ratio of CD11b⁺Siglec-H⁺ cells to lymphocytes in the nasal mucosa herein means the ratio of CD11b⁺Siglec- H⁺ cells to all lymphocytes contained in the nasal mucosa. The ratio of CD11b⁺Siglec-H⁺ cells to lymphocytes in the nasal mucosa is not limited, but can be calculated as the ratio (percentage) of CD11b⁺Siglec-H⁺ cells to all lymphocytes in the lymphocyte fraction in analysis using FACS, as shown in, for instance, the Examples below.

In the present invention, the wording "increase the ratio of CD11b⁺Siglec-H⁺ cells to lymphocytes in the nasal mucosa" means an increase in the ratio of CD11b⁺Siglec-H⁺ cells to lymphocytes in the nasal mucosa when the lactic acid bacterium is administered, compared to the corresponding ratio when the lactic acid bacterium is not administered. Specifically, when the ratio of CD11b⁺Siglec-H⁺ cells to lymphocytes in the nasal mucosa of a subject after administration of the lactic acid bacterium is, for example, 1.1-fold, 1.2-fold, 1.3-fold, 1.4-fold, 1.5-fold, 1.6-fold, 1.7-fold, 1.8-fold, 1.9-fold, 2.0-fold, 2.1-fold, 2.2-fold, 2.3-fold, 2.4-fold, 2.5-fold, 2.6-fold, 2.7-fold, 2.8-fold, 2.9-fold, 3.0-fold, 3.1-fold, 3.2-fold, 3.3-fold, 3.4-fold, 3.5-fold, 3.6-fold, 3.7-fold, 3.8-fold, 3.9-fold, 4.0-fold, 4.1-fold, 4.2-fold, 4.3-fold, 4.4-fold, 4.5-fold, 4.6-fold, 4.7-fold, 4.8-fold, 4.9-fold, or 5.0-fold or more higher than the corresponding ratio in the subject before administration to the subject, the ratio of CD11b⁺Siglec-H⁺ cells to lymphocytes in the nasal mucosa can be said to have increased.

The pharmaceutical composition of the present invention may be administered to mammals. The mammals are not limited, but include, for example, primates, rodents, or carnivores, and are preferably primates. Examples of the primates include humans, chimpanzees, rhesus monkeys, marmosets, canines, felines, bovines, equines, porcines, and ovines, and the primates are preferably humans.

The pharmaceutical composition of the present invention may be administered either before contracting or the onset of an infectious disease (or before infection with a pathogen) or after contracting or the onset of an infectious disease (or after infection with a pathogen). When used for the prevention of infectious diseases, administration before infection is desirable, but administration may be early after infection (e.g., within 5 days after infection) to prevent aggravation of disease after infection, and the timing of administration may be determined by taking into consideration the prevalence of the viral infection and the time of year when the disease is likely to spread.

The number of doses of the pharmaceutical composition of the present invention may be optionally set according to the purpose of administration and the traits, physical conditions, symptoms, etc., of the subject of administration, but can be one or more times (e.g., two or more times, three or more times, four or more times, five or more times). The dosing interval for multiple doses may be 1 to 24 hours or 1 to 10 days, e.g., every 1 day, 2 days, 3 days, 4 days, or 5 days. When used for the prevention or treatment of infectious diseases, it is preferable that the administration is twice or more times while the dosing interval is every 1 day to maintain the prophylactic or therapeutic effects and to reduce medical costs.

When the pharmaceutical composition of the present invention is used for the prevention of viral infections, the beginning of the effective period during which the active ingredient produces a prophylactic effect is, in terms of immediate effect, day 0, day 1, day 2, day 3, day 4, day 5, day 6, or day 7 from the last day of administration.

When the pharmaceutical composition of the present invention is used for the prevention of viral infections, the end of the effective period during which the active ingredient produces a prophylactic effect is, in terms of maintaining the effect, day 7, day 14, day 28, or day 56 from the last day of administration.

For the pharmaceutical composition of the invention, the above beginning and end dates may be optionally combined, and may be, for example, from day 0 to day 56, from day 0 to day 28, from day 0 to day 14, from day 0 to day 7, from day 3 to day 56, from day 3 to day 28, from day 3 to day 14, from day 3 to day 7, from day 5 to day 56, from day 5 to day 28, from day 5 to day 14, from day 5 to day 7, from day 7 to day 56, from day 7 to day 28, or from day 7 to day 14.

When the pharmaceutical composition of the present invention is used for the prevention of viral infections, the effective period during which the active ingredient produces a prophylactic effect is within 56 days, 28 days, 14 days, 7 days, 6 days, 5 days, 4 days, or 3 days from the last day of administration.

The daily dose of the active ingredient in the present invention may be adjusted and determined, if appropriate, according to the body weight of the subject of administration. In the case of an adult (e.g., human adult), based on 50 kg body weight, the following limits can be specified by, for example, the dried bacterial cell mass, and in terms of infection prevention or infection suppression effects, the lower limit (or more or more than) may be 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, or 50 mg, and the upper limit (or less or less than) may be 1000 mg, 950 mg, 900 mg, 850 mg, 800 mg, 750 mg, 700 mg, 650 mg, 600 mg 550 mg, 500 mg, 475 mg, 450 mg, 425 mg, 400 mg, 375 mg, 350 mg, 325 mg, 300 mg, 275 mg, 250 mg, 225 mg, 200 mg, 190 mg, 180 mg, 170 mg, 160 mg, 150 mg, 140 mg, 130 mg, 120 mg, 110 mg or 100 mg, 90 mg, 80 mg, 70 mg, 60 mg, or 50 mg. These upper and lower limits can be optionally combined, and the above dose ranges may be, for example, 1 mg to 1000 mg, 10 mg to 500 mg, 25 mg to 450 mg, 30 mg to 400 mg, 35 mg to 375 mg, 40 mg to 350 mg, 40 mg to 325 mg, 45 mg to 300 mg, 45 mg to 250 mg, 45 mg to 200 mg, 45 mg to 150 mg, 45 mg to 100 mg, 45 mg to 90 mg, 45 mg to 80 mg, 45 mg to 70 mg, or 45 mg to 60 mg.

The daily dose of the active ingredient in the present invention can also be specified by the number of bacteria, and in the case of an adult (e.g., human adult), based on 50 kg body weight, the lower limit (or more or more than) may be 1 × 10⁸, 1 × 10⁹, 1 × 10¹⁰, or 1 × 10¹¹ cells and the upper limit (or less or less than) may be 1 × 10¹⁴, 1 × 10¹¹, 1 × 10¹², 5 × 10¹¹, or 1 × 10¹¹ cells in terms of infection prevention or infection suppression effects. These upper and lower limits may each be optionally combined, and the above dose ranges may be, for example, 1 × 10⁸ to 1 × 10¹⁴, 1 × 10⁹ to 1 × 10¹³, 1 × 10¹⁰ to 1 × 10¹², or 5 × 10¹⁰ to 5 × 10¹¹ or 1 × 10¹¹. The number of lactic acid bacterium cells can be measured, for example, by fluorescent staining, flow cytometry, or culture methods, and from the viewpoint of accuracy, measurement by flow cytometry is preferable.

It is preferable to continue the administration of the active ingredient in the present invention for a period during which the infection prevention or infection suppression effects are expected. The period of administration of the active ingredient in the present invention may be, for example, one week or more, two weeks or more, three weeks or more, or one month or more (four weeks or more) at the above daily dose from the viewpoint of producing better infection prevention or infection suppression effects. The dosing interval of the active ingredient in the present invention may be once every three days, once every two days, or once a day at the above daily dose, and is preferably once a day.

Administration of the active ingredient of the invention may also start prior to the event where or time when prevention of infection is expected, i.e., when the likelihood of contracting or developing an infectious disease (the likelihood of infection with a pathogen) is increased. Examples of the event where the infection-preventive effect is expected include a behavior that increases the likelihood of contracting or developing an infectious disease (the likelihood of infection with a pathogen) (e.g., participation in high-risk events, travel to endemic areas, infection of family members or others living together), and examples of the time when the infection-preventive effect is expected include the period of infectious disease outbreaks. Examples of the timing of administration (administration timing) prior to the event where the infection-preventive effect is expected include at least one day, three days, one week, two weeks, three weeks, one month (four weeks or more), or two months (eight weeks or more) prior to the event. In addition, although not particularly limited, the active ingredient may be taken continuously from the start of administration until the event where the infection-preventive effect is expected, and in some cases may be taken with dosing intervals. In the present invention, the preferred timing of administration prior to the event where the infection-preventive effect is expected may be, for example, total two or more times at 1 day and 3 days before, total two or more times at 4 days and 6 days before, total two or more times at 7 days and 9 days before, total two or more times at 10 days and 12 days before, or total two or more times at 14 days and 16 days before the event.

Administration of the active ingredient in the invention may also start after the event where or time when the infection-preventive effect is expected. Examples of the administration timing after the event where the infection-preventive effect is expected include one or more days after, three or more days after, one or more weeks after, or two or more weeks after the event. In addition, although not particularly limited, the active ingredient may be taken continuously with dosing intervals in some cases when the administration is performed after the event where the infection-preventive effect is expected. In the present invention, particularly preferably, the administration of the active ingredient in the invention may be started prior to the event where the infection-preventive effect is expected and the administration may continue until after the event.

The administration of the active ingredient in the present invention may be combined with a known infection prevention or infection suppression method. Examples of the known infection prevention or infection suppression method include administration of an immunostimulant (e.g., fucoidan, polysaccharides such as β-glucan, lactoferrin, propolis, bifidobacteria), administration of antiviral agents (e.g., M2 ion channel inhibitors (e.g., amantadine or rimantadine), neuraminidase inhibitors (oseltamivir or zanamivir), and their salts), administration of neutralizing antibody medicines (tixagevimab and cilgavimab), and administration of vaccines (e.g., live, inactivated, and recombinant protein vaccines, mRNA (messenger RNA) vaccines, DNA vaccines, viral vector vaccines).

Another aspect of the present invention provides a method of preventing or a method of treating a viral infectious disease, a method of reducing a risk of contracting an infectious disease or a method of reducing a risk of infection with a pathogen, or a method of immunostimulation, the method comprising the step of topically administering an effective amount of lactic acid bacterium or a composition comprising the lactic acid bacterium to a subject in need thereof. The method of the present invention can be carried out according to the description for the pharmaceutical composition of the present invention.

Another aspect of the present invention also provides use of a lactic acid bacterium for the manufacture of a viral infectious disease prophylactic or therapeutic agent for topical administration, for the manufacture of an infectious disease contraction risk reducing agent or a pathogen infection risk reducing agent for topical administration, or for the manufacture of an immunostimulant for topical administration. The present invention also provides use of a lactic acid bacterium as a viral infectious disease prophylactic or therapeutic agent for topical administration, as an infectious disease contraction risk reducing agent or a pathogen infection risk reducing agent for topical administration, or as an immunostimulant for topical administration. The present invention also provides use of a lactic acid bacterium in a method of preventing or treating an infectious disease by topical administration, in a method of reducing a risk of contracting an infectious disease or a risk of infection with a pathogen by topical administration, or in a method of immunostimulation by topical administration. The use of the present invention can be implemented according to the description for the pharmaceutical composition of the present invention and the method of the present invention.

Another aspect of the present invention also provides a lactic acid bacterium for use in viral infectious disease prevention or treatment by topical administration, for use in reducing a risk of contracting an infectious disease or a risk of infection with a pathogen by topical administration, or for use in immunostimulation by topical administration. The lactic acid bacterium of the present invention can be implemented according to the description for the pharmaceutical composition of the present invention and the method of the present invention.

### Examples

Hereinafter, the present invention is further described in detail based on the following Examples and others, however, the present invention is not limited to these Examples.

### Example 1: Study on effect of preventing SARS-CoV-2 infection by intranasal administration (1)

In Example 1, the effect of the lactic acid bacterium on prevention of SARS-CoV-2 infection by intranasal administration was studied.

### (1) Method

### A. Administration of lactic acid bacterium and SARS-CoV-2 infection

BALB/c mice (17 weeks old, female, body weight: about 20 g, Japan SLC, Inc.) were divided into total 6 groups: a control group (lactic acid bacterium non-administration group) and 5 lactic acid bacterium administration groups, and heat-killed lactic acid bacteria *(Lactococcus lactis* subsp. *lactis* JCM5805 strain) were administered under the conditions shown in Table 1 (the number n of each group was 2 or 3). Mice in each group were infected, under anesthesia on day 0 of infection, with the mouse-adapted SARS-CoV-2 QHmusX strain (Iwata-Yoshikawa N et al., Science Advances, (2022) 8(1): eabh3827) established at the National Institute of Infectious Diseases (NIID) at 0.5 × LD₅₀ (30 µl). They were also weighed before and 3 days after infection. Then, on day 3 after infection, they were dissected and the lungs and bronchoalveolar lavage fluid (BALF) were collected.

### [Table 1]

**Table 1: Lactic acid bacterium administration conditions**

| | Timing of administration* | Dose (per animal)** |
|---|---|---|
| Control group (non-administration group) | - | - |
| Day (-1/-3) group | 1 day before infection and 3 days before infection | 1 mg/day |
| Day (-2/-4) group | 2 days before infection and 4 days before infection | 1 mg/day |
| Day (-3/-5) group | 3 days before infection and 5 days before infection | 1 mg/day |
| Day (-1/-3) (low) group | 1 day before infection and 3 days before infection | 0.2 mg/day |
| Day (-2/-7) group | 2 days before infection and 7 days before infection | 1 mg/day |
| Day (-3/-8) group | 3 days before infection and 8 days before infection | 1 mg/day |

| | | |
|---|---|---|
| * Timing of administration indicates each corresponding date before SARS-CoV-2 infection. ** Dose: 0.2 mg/day or 1 mg/day was administered intranasally under anesthesia by using a lactic acid bacterium liquid (50 µl) prepared at 4 mg/ml or 20 mg/ml, respectively, in PBS (GIBCO). | | |

### B. To extract and prepare RNA

The collected lungs were each immersed in TRIzol (Thermo Fisher Scientific) and dispersed in a gentleMACS M tube (Miltenyi Biotec). After centrifugation, the supernatant was collected and chloroform (Nacalai Tesque) was added, and after centrifugation, the upper aqueous layer was collected into a tube containing 2-propanol (Nacalai Tesque). After centrifugation, the supernatant was removed; the pellet was suspended in 75% ethanol; the suspension was centrifuged; the supernatant was completely removed; and the rest were dried, and then suspended in RNase-Free Water (GIBCO). RNA from the collected BALF was also prepared using a QIAmp ViralRNA Mini Kit (QIAGEN).

### C. To quantify RNA

The extracted RNA was quantified by real-time PCR. The real-time PCR was performed using a QuantiTect Probe RT-PCR Kit (QIAGEN) and gene-specific SARS-CoV-2 primers/probes, and the reaction was carried out according to the common reaction composition at 50°C for 30 minutes, 95°C for 15 minutes, followed by 45 cycles of 95°C for 15 seconds and 60°C for 60 seconds. The primers used were those listed in Table 2 (Shirato K., et al., Jpn. J. Infect. Dis. 2020; 73(4): 304-307).

### [Table 2]

**Table 2: Primers**

| Primer name | Sequence (5' to 3') |
|---|---|
| NIID_2019-nCOV_N_F2 | AAATTTTGGGGACCAGGAAC |
| NIID_2019-nCOV_N_R2 | TGGCAGCTGTGTAGGTCAAC |
| NIID_2019-nCOV_N_P2 | FAM-ATGTCGCGCATTGGCATGGA-BHQ |

### D. To quantify virus

Infectious SARS-CoV-2 virus in BALF was quantified. Specifically, a 10-fold (10 to 10⁶) step dilution series was prepared using DMEM (GIBCO) supplemented with 10 mass% FCS, and Vero cells (African green monkey kidney cell line (JCRB1819 VeroE6/TMPRSS2, JCR B cell bank)) were seeded onto a 96-well plate (CORNING); and 100 µl of each BALF dilution was inoculated in 4 wells, and the viral infection was evaluated by the presence or absence of cell degeneration at 4 days after inoculation. The viral titer was calculated as TCID₅₀/ml according to the Reed-Muench formula.

### (2) Results

The results are as shown in Figs. 1 to 3. Fig. 1 shows that based on the percentage of the body weight on day 3 of infection (approximately 72 hours after infection) to the body weight before SARS-CoV-2 infection, no weight loss was observed in any of the lactic acid bacterium administration groups, but the control group (lactic acid bacterium non-administration group) showed that the body weight decreased to about 90% of the body weight before infection. The weight loss suppression effect was also observed in the Day (-2/-7) and Day (-3/-8) groups, indicating that the infection suppression effect by intranasal administration of the lactic acid bacterium was maintained for several days (at least 3 days). In addition, in the control group, it was observed that the mice were less hairy and less active, and had a lower number of diet intakes than those of the lactic acid bacterium administration groups.

In Fig. 2, it was found that the level of SARS-CoV-2 RNA (copy number) in the lungs or BALF at 3 days after SARS-CoV-2 infection (approximately 72 hours after infection), compared to the control group, was significantly decreased in Day (-1/-3), Day (-2/-4), and Day (-3/-5) groups, and was decreased in Day (-1/-3) (low), Day (-2/-7), and Day (-3/-8) groups. The decrease in RNA level was also detected in the Day (-2/-7) and Day (-3/-8) groups, indicating that the infection suppression effect by intranasal administration of the lactic acid bacterium was maintained for several days (at least 3 days).

In Fig. 3, it was found that the viral load of infectious SARS-CoV-2 in the BALF at 3 days after SARS-CoV-2 infection (approximately 72 hours after infection), compared to the control group, was significantly decreased in Day (-1/-3), Day (-2/-4), and Day (-3/-5) groups, and was decreased in Day (-1/-3) (low), Day (-2/-7), and Day (-3/-8) groups. The decrease in viral load was also detected in the Day (-2/-7) and Day (-3/-8) groups, indicating that the infection suppression effect by intranasal administration of the lactic acid bacterium was maintained for several days (at least 3 days).

These results have demonstrated that administration of the lactic acid bacterium not only suppresses infection, but also produces effect of suppressing aggravation of the disease.

### Example 2: Study on effect of preventing SARS-CoV-2 infection by intranasal administration (2)

In Example 2, the effect of intranasal administration of the lactic acid bacterium on IFN-α production in blood or BALF after SARS-CoV-2 infection was studied.

### (1) Method

BALB/c mice (17 weeks old, female, body weight: about 20 g, Japan SLC, Inc.) were divided into total 6 groups: a control group (lactic acid bacterium non-administration group) and 5 lactic acid bacterium administration groups, and heat-killed lactic acid bacteria *(Lactococcus lactis* subsp. *lactis* JCM5805 strain) were administered under the conditions shown in Table 3 (the number n of each group was 2 or 3). Mice in each group were infected, under anesthesia on day 0 of infection, with the mouse-adapted SARS-CoV-2 QHmusX strain (Iwata-Yoshikawa N et al., Science Advances, (2022) 8(1): eabh3827) established at the National Institute of Infectious Diseases (NIID) at 0.5 × LD₅₀ (30 µl). Then, on day 3 (approximately 72 hours) after infection, the mice were dissected and interferon-a (IFN-α) levels in blood and bronchoalveolar lavage fluid (BALF) were measured using an interferon α All Subtype ELISA Kit (PBL BIOMEDICAL LABORATORIES).

### [Table 3]

**Table 3: Lactic acid bacterium administration conditions**

| | Timing of administration* | Dose (per animal)** |
|---|---|---|
| Control group (non-administration group) | - | - |
| Day (-1) group | 1 day before infection | 1 mg/day |
| Day (-1/-2) group | 1 day before infection and 2 days before infection | 1 mg/day |
| Day (-1/-3) group | 1 day before infection and 3 days before infection | 1 mg/day |
| Day (-1/-2/-3/-4) group | 1 day before infection, 2 days before infection, 3 days before infection, 4 days before infection | 1 mg/day |

| | | |
|---|---|---|
| * Timing of administration indicates each corresponding date before SARS-CoV-2 infection. ** Dose: 0.2 mg/day or 1 mg/day was administered intranasally under anesthesia by using a lactic acid bacterium liquid (50 µl) prepared at 4 mg/ml or 20 mg/ml, respectively, in PBS (GIBCO). | | |

### (2) Results

The results are as shown in Fig. 4. It was found that in the control group, the average IFN-α level in blood at 3 days after SARS-CoV-2 infection (approximately 72 hours after infection) exceeded 100 pg/ml, and the average IFN-α level in BALF exceeded 300 pg/ml. By contrast, it was found that in the lactic acid bacterium administration groups, the average IFN-α level in blood at 3 days after SARS-CoV-2 infection (approximately 72 hours after infection) was 50 pg/ml or less, and the average IFN-α level in BALF was 200 pg/ml or less.

### Example 3: Study on effect of preventing SARS-CoV-2 infection by intranasal administration (3)

In Example 3, the effect of the lactic acid bacterium on suppressing a body weight loss after SARS-CoV-2 infection by intranasal administration was studied.

### (1) Method

BALB/c mice (16 weeks old, female, body weight: about 20 g, Japan SLC, Inc.) were divided into 2 groups: a control group (lactic acid bacterium non-administration group) and a lactic acid bacterium administration group, and heat-killed lactic acid bacteria *(Lactococcus lactis* subsp. *lactis* JCM5805 strain) were administered under the conditions shown in Table 4 (the number n of each group was 6). Mice in each group were infected, under anesthesia on day 0 of infection, with the mouse-adapted SARS-CoV-2 QHmusX strain (Iwata-Yoshikawa N et al., Science Advances, (2022) 8(1): eabh3827) established at the National Institute of Infectious Diseases (NIID) at 0.5 × LD₅₀ (30 µl). The mice were then weighed daily until day 7 after infection, and the percentage of the body weight to the body weight before infection was calculated.

### [Table 4]

**Table 4: Lactic acid bacterium administration conditions**

| | Timing of administration* | Dose (per animal)** |
|---|---|---|
| Control group (non-administration group) | - | - |
| Lactic acid bacterium administration group | 1 day before infection and 3 days before infection | 0.2 mg/day |

| | | |
|---|---|---|
| *Timing of administration indicates each date before SARS-CoV-2 infection. **Dose of 0.2 mg/day was administered intranasally under anesthesia by using a lactic acid bacterium liquid (50 µl) prepared at 4 mg/ml in PBS (GIBCO). Note that the control group received lactic acid bacterium-free PBS (50 µl) intranasally under anesthesia. | | |

### (2) Results

The results are as shown in Fig. 5. The control group showed a significant weight loss, peaking 3 to 4 days after infection. By contrast, the lactic acid bacterium administration group showed no obvious body weight loss after infection and a significantly suppressed body weight loss compared to the control group.

### Example 4: Study on effect of preventing SARS-CoV-2 infection by intranasal administration (4)

In Example 4, the effect of the lactic acid bacterium on maintaining the SARS-CoV-2 infection preventive effect by intranasal administration was studied.

### (1) Method

### A. Administration of lactic acid bacterium and SARS-CoV-2 infection

BALB/c mice (15 weeks old, female, body weight: about 20 g, Japan SLC, Inc.) were divided into total 6 groups: a control group (lactic acid bacterium non-administration group) and 5 lactic acid bacterium administration groups, and heat-killed lactic acid bacteria *(Lactococcus lactis* subsp. *lactis* JCM5805 strain) were administered under the conditions shown in Table 5 (the number n of each group was 4 or 5). Mice in each group were infected, under anesthesia on day 0 of infection, with the mouse-adapted SARS-CoV-2 QHmusX strain (Iwata-Yoshikawa N et al., Science Advances, (2022) 8(1): eabh3827) established at the National Institute of Infectious Diseases (NIID) at 5 × LD₅₀ (5 µl). Two days after infection, the mice were then dissected and nasal-associated lymphoid tissues (NALT) were collected.

### [Table 5]

**Table 5: Lactic acid bacterium administration conditions**

| | Timing of administration* | Dose (per animal)** |
|---|---|---|
| Control group (non-administration group) | - | - |
| Day (-1/-3) group | 1 day before infection and 3 days before infection | 1 mg/day |
| Day (-4/-6) group | 4 days before infection and 6 days before infection | 1 mg/day |
| Day (-7/-9) group | 7 days before infection and 9 days before infection | 1 mg/day |
| Day (-10/-12) group | 10 days before infection and 12 days before infection | 1 mg/day |
| Day (-14/-16) group | 14 days before infection and 16 days before infection | 1 mg/day |

| | | |
|---|---|---|
| *Timing of administration indicates each corresponding date before SARS-CoV-2 infection. **Dose of 1 mg/day was administered intranasally under anesthesia by using a lactic acid bacterium liquid (10 µl) prepared at 100 mg/ml in PBS (Gibco). Note that the control group received lactic acid bacterium-free PBS (10 µl) intranasally under anesthesia. | | |

### B. To extract and prepare RNA

RNA was extracted and prepared from the collected NALT in the same manner as described in Example 1(1)B.

### C. To quantify RNA

The RNA was quantified as described in Example 1(1)C.

### (2) Results

The results are as shown in Fig. 6. The level of SARS-CoV-2 viral RNA and the level of subgenomic RNA were significantly lower in the Day (-1/-3) and Day (-4/-6) groups than the control group. In addition, the Day (-7/-9), Day (-10/-12), and Day (-14/-16) groups also tended to have approximately 10-fold lower viral and subgenomic RNA levels than the control group.

### Example 5: Study on effect of preventing influenza A virus infection by intranasal administration

In Example 5, the effect of the lactic acid bacterium on prevention of influenza A virus (H1N1) infection by intranasal administration was studied.

### (1) Method

### A. Administration of lactic acid bacterium and influenza A virus infection

BALB/c mice (12 weeks old, female, body weight: about 20 g, Japan SLC, Inc.) were divided into 2 groups: a control group (lactic acid bacterium non-administration group) and a lactic acid bacterium administration group, and heat-killed lactic acid bacteria *(Lactococcus lactis* subsp. *lactis* JCM5805 strain) were administered under the conditions shown in Tables 6 and 7 (the number n of each group was 5 or 7). Mice in each group in Tables 6 and 7 were anesthetized on day 0 of infection and were each infected with influenza A virus A/PR8/34 (PR8) (obtained from the National Institute of Infectious Diseases; Ikeda K, Ainai A, et al., Vaccine, 2015; 33 (45): 6066-9) or A/Narita/1/2009 (Narita) (obtained from the National Institute of Infectious Diseases; Adachi Y, et al., J Exp Med., 2015, 212 (10): 1709-23) at 40 × LD₅₀ (5 µl) or 5 × LD₅₀ (5 µl), respectively. The mice were then dissected 2 days after infection and nasal lavage fluid was collected.

### [Table 6]

**Table 6: Lactic acid bacterium administration conditions (PR8 infection)**

| | Timing of administration* | Dose (per animal)** |
|---|---|---|
| Control group (non-administration group) | - | - |
| Lactic acid bacterium administration group | 1 day before infection and 3 days before infection | 1 mg/day |

| | | |
|---|---|---|
| *Timing of administration indicates days before SARS-CoV-2 infection. **Dose of 1 mg/day was administered intranasally under anesthesia by using a lactic acid bacterium liquid (10 µl) prepared at 100 mg/ml in PBS (Gibco). Note that the control group received lactic acid bacterium-free PBS (10 µl) intranasally under anesthesia. | | |

### [Table 7]

**Table 7: Lactic acid bacterium administration conditions (Narita infection)**

| | Timing of administration* | Dose (per animal)** |
|---|---|---|
| Control group (non-administration group) | - | - |
| Lactic acid bacterium administration group | 1 day before infection and 3 days before infection | 1 mg/day |

| | | |
|---|---|---|
| *Timing of administration indicates days before SARS-CoV-2 infection. **Dose of 1 mg/day was administered intranasally under anesthesia by using a lactic acid bacterium liquid (10 µl) prepared at 100 mg/ml in PBS (Gibco). Note that the control group received lactic acid bacterium-free PBS (10 µl) intranasally under anesthesia. | | |

### B. To extract and prepare RNA

RNA from the collected nasal lavage fluid was also prepared using a QIAmp ViralRNA Mini Kit (QIAGEN).

### C. To quantify RNA

The extracted RNA was quantified by real-time PCR. The real-time PCR was performed using a QuantiTect Probe RT-PCR Kit (QIAGEN) and gene-specific influenza A virus primers/probes, and the reaction was carried out according to the common reaction composition at 50°C for 30 minutes, 95°C for 15 minutes, followed by 40 cycles of 94°C for 15 seconds and 56°C for 75 seconds. The primers used were those shown in Table 8 (Nakauchi M, et al., Journal of Virological Methods, 2011, 7171(1): 156-162).

### [Table 8]

**Table 8: Primers**

| Primer name | Sequence (5' to 3') |
|---|---|
| MP-39-67_forward | CCMAGGTCGAAACGTAYGTTCTCTCTATC |
| MP-183-153_reverse | TGACAGRATYGGTCTTGTCTTTAGCCAYTCCA |
| MP-96-75ProbeAs | ATYTCGGCTTTGAGGGGGCCTG |

### (2) Results

The results are as shown in Fig. 7. The lactic acid bacterium administration group had a significantly lower level of influenza A viral RNA in nasal lavage fluid for both PR8 and Narita infections than the control group.

### Example 6: Study on immunostimulatory effect by intranasal administration (1)

In Example 6, the effect of the lactic acid bacterium on immunostimulation by intranasal administration was studied.

### (1) Method

### A. Administration of lactic acid bacterium

BALB/c mice (16 weeks old, female, body weight: about 20 g, Japan SLC, Inc.) were divided into 4 groups shown in Table 9, and a lactic acid bacterium intranasal administration study was conducted. The control group received PBS (GIBCO) and the administration group received heat-killed lactic acid bacteria (*Lactococcus lactis* subsp. *lactis* JCM5805 strain) under the conditions shown in Table 9 (the number n of each group was 3). Note that the control group received only PBS corresponding to the dose administered to the administration group. Then, on day 3 after administration of lactic acid bacterium, the mice were euthanized and dissected by taking a complete blood sample from the heart under anesthesia.

### [Table 9]

**Table 9: Lactic acid bacterium intranasal administration conditions**

| Group*¹ | Date*² of administration of PBS or lactic acid bacterium liquid | Dose volume of PBS or lactic acid bacterium liquid per animal (Dose of lactic acid bacterium) |
|---|---|---|
| Low Volume control group (Low Volume (-) group) | 3 days before dissection (Day (-3)) and 1 day before dissection (Day (-1)) | 10 µl (-) |
| Low Volume administration group (Low Volume (+) group) | 3 days before dissection (Day (-3)) and 1 day before dissection (Day (-1)) | 10 µl (1 mg/day) |
| High Volume control group (High Volume (-) group) | 3 days before dissection (Day (-3)) and 1 day before dissection (Day (-1)) | 50 µl (-) |
| High Volume administration group (High Volume (+) group) | 3 days before dissection (Day (-3)) and 1 day before dissection (Day (-1)) | 50 µl (0.2 mg/day) |

| | | |
|---|---|---|
| *¹ In the Low Volume (-) and High Volume (-) groups, 10 µl or 50 µl of PBS (free of any lactic acid bacterium) was each administered intranasally. In the Low Volume (-) and High Volume (+) groups, 10 µl or 50 µl of lactic acid bacterium liquid, which had been prepared at 100 mg/ml or 4 mg/ml, respectively, in PBS, was administered intranasally under anesthesia. Note that the + or - indicates the presence or absence of lactic acid bacterium administration. *² Day of administration is indicated by each date counting from the day of dissection (day 0). | | |

### B. To extract RNA

RNA was extracted from each tissue sample by using the following procedures (i) to (vi).
(i) Each tissue sample was collected in M-tubes (Miltenyi Biotec) containing 1-ml TRIzol (Thermo Fisher Scientific) and cryopreserved at -80°C.
(ii) The cryopreserved tissue samples were each thawed and the tissue was crushed using GentleMACS (Miltenyi Biotec), allowed to stand at room temperature for 2 to 3 minutes, and then centrifuged at 3,000 × g for 5 minutes at 4°C. The supernatant was collected in an Eppendorf tube and further centrifuged at 12,000 × g for 10 minutes at 4°C.
(iii) The supernatant was collected in an Eppendorf tube and 180 µl of chloroform (FUJIFILM Wako Pure Chemical Corporation) was added; the mixture was allowed to stand at room temperature for 2-3 minutes, and then centrifuged at 12,000 × g for 15 minutes at 4°C.
(iv) The aqueous layer at the top of the supernatant was collected in an Eppendorf tube containing 450 µl of 2-propanol (FUJIFILM Wako Pure Chemical Corporation), allowed to stand at room temperature for 10 minutes, and then centrifuged at 12,000 × g for 10 minutes at 4°C.
(v) The supernatant was removed and 1 ml of 75% ethanol (FUJIFILM Wako Pure Chemical Corporation) was added; the mixture was vortexed, and centrifuged at 7,500 × g for 5 minutes at 4°C.
(vi) The supernatant was removed and the pellet was air-dried for 15 minutes. The resulting material was then suspended in RNase Free Water (QIAGEN), heat-treated at 56°C for 10 minutes, and stored at - 80°C.

### C. To quantify RNA

The extracted RNA was used to quantify the mRAN expression level of each IFN-inducible antiviral gene (ISG): Viperin, Isg15, or Mx1. Specifically, the following procedures (i) to (v) were used.
(i) The RNA concentration of each sample was measured by Nanodrop and then diluted with Nuclease Free Water in the iScript cDNA synthesis kit (BIORAD) to 1000 ng/15 µl.
(ii) MasterMix (5 × iScript reaction mix: 4 µl/sample and iScript reverse transcript: 1 µl/sample) was prepared and 5 µl each was added to each RNA solution.
(iii) cDNA was then synthesized in a thermal cycler and stored at -20°C.
(iv) Each cDNA sample was diluted 5-fold with MilliQ water, as well as standard solutions mixed with all samples were prepared at concentrations of ×1, ×2, ×4, ×8, ×16, ×32, ×64, and ×128.
(v) Reaction solutions shown in Table 10 were applied to a 96-well plate, and real-time PCR was performed using a Light Cycler 480 for 45 cycles of a set of reactions at 95°C for 10 seconds, 60°C for 10 seconds, and 72°C for 10 seconds. The primers used were those listed in Table 11.

### [Table 10]

**Table 10: Composition of reaction solution**

| | |
|---|---|
| TB Green Premix Ex TaqII (Takara Bio Inc.) | 10 µl |
| Forward primer | 0.8 µl |
| Reverse primer | 0.8 µl |
| MilliQ | 6.4 µl |
| cDNA | 2.0 µl |
| Total volume | 20 µl |

### [Table 11]

**Table 11: Primers**

| Primer name | Sequence (5' to 3') |
|---|---|
| Viperin_forward | CCCGTGAGTGTCAACTACCAC |
| Viperin_reverse | GCCCAAGTATTCACCCCTGTC |
| Isg15_forward | GGTGTCCGTGACTAACTCCAT |
| Isg15_reverse | CTGTACCACTAGCATCACTGTG |
| Mx1_forward | GACCATAGGGGTCTTGACCAA |
| Mx1_reverse | AGACTTGCTCTTTCTGAAAAGCC |
| Gapdh_forward (internal standard) | AGGTCGGTGTGAACGGATTTG |
| Gapdh reverse (internal standard) | GGGGTCGTTGATGGCAACA |

### (2) Results

The results are as shown in Figs. 8 to 11. In Fig. 8, from the percentage of the body weight on Day (-2) (2 days before dissection), Day (-1) (1 day before dissection (day of second intranasal administration of lactic acid bacterium)), or Day (0) (day of dissection) relative to the body weight on Day (-3) (3 days before dissection (day of the first intranasal administration of lactic acid bacterium)), Day (0) (day of dissection) was compared between the Low Volume (-) and Low Volume (+) groups, and no significant differences were found. There was no significant difference between the High Volume (-) group and the High Volume (+) group on Day (0) (the day of dissection). In addition, no effects on their hairiness or activity were observed in any of the groups.

In Fig. 9, the Low Volume (+) group showed a significant increase in Viperin and Isg15 mRNA expression levels in the submandibular lymph nodes compared to the Low Volume (-) group, as well as a trend toward an increased Mx1 mRNA expression level. By contrast, the High Volume (+) group showed no significant increase compared to the High Volume (-) group. The Low Volume (+) and the High Volume (+) had a difference in the volume of lactic acid bacterium liquid administered, so that they are applied to the nasal cavity and lungs, respectively. The results have suggested that the nasal cavity is the preferred site for topical administration.

In Fig. 10, the Low Volume (+) group showed a significant increase in Viperin and Isg15 expression levels in the spleen compared to the Low Volume (-) group, as well as a trend toward an increased Mx1 expression level. By contrast, the High Volume (+) group showed no significant increase compared to the High Volume (-) group.

In Fig. 11, the Low Volume (+) group showed a trend toward an increased expression level of Isg15 in the lungs compared to the Low Volume (-) group.

### Example 7: Study on immunostimulatory effect by intranasal administration (2)

In Example 7, how intranasal administration of the lactic acid bacterium affected pDC was studied using the spleen, submandibular lymph nodes, and nasal mucosa excised by dissection in Example 6.

### (1) Method

The number of pDCs in each tissue: the spleen, submandibular lymph nodes, or nasal mucosa excised by dissection in Example 6 was quantified using the following procedure. Each tissue was stained using the fluoresce-labeled antibodies listed in Table 12, and the stained cells were washed once with FACS buffer, resuspended in FACS buffer, and subjected to FACS analysis. Data was then acquired using FACS Cant II (BD Biosciences) and analyzed using FlowJo software (Tree Star). Here, cells expressing CD11c and Siglec-H (CD11c⁺Siglec-H⁺ cells) or cells expressing Siglec-H but without expressing CD11b (CD11b⁻Siglec-H⁺ cells) were defined as pDC, the ratio of pDC to lymphocytes in each tissue was quantified from the ratio of pDC to all lymphocytes contained in the lymphocyte fraction of the FACS analysis, and the expression levels of MHC class II (I-A/I-E) and CD86 were measured as cell surface markers for pDC activation. In addition, the ratio of CD11b⁺Siglec-H⁺ cells to lymphocytes in each tissue was quantified from the ratio of cells expressing CD11b and Siglec-H (CD11b⁺Siglec-H⁺ cells) contained in the lymphocyte fraction of the FACS analysis.

### [Table 12]

**Table 12: Fluorescence-labeled antibodies**

| |
|---|
| anti-mouse I-A/I-E Antibody-FITC (clone:M5 / 114.15.2, BioLegend Inc.) |
| anti-mouse CD86 Antibody-PE (clone:GL1, BioLegend Inc.) |
| anti-mouse Siglec-H Antibody-APC (clone:551, BioLegend Inc.) |
| anti-mouse CD11c Antibody-PE-Cy7 (clone:N418, BioLegend Inc.) |
| anti-mouse CD11b Antibody-APC/Cy7 (clone:M1/70, BioLegend Inc.) |

### (2) Results

The results are as shown in Figs. 12 to 17. In Fig. 12, the ratio of pDC to lymphocytes in the spleen tended to increase in the Low Volume administration group (Low Volume (+) group) compared to the Low Volume control group (Low Volume (-) group). On the other hand, Fig. 13 shows no significant differences in the expression levels of MHC class II (I-A/I-E) (Fig. 13A) and CD86 (Fig. 13B) on pDC in the spleen.

In Fig. 14, the ratio of pDC to lymphocytes in the submandibular lymph nodes was significantly decreased in the Low Volume administration group (Low Volume (+) group) compared to the Low Volume control group (Low Volume (-) group) (p < 0.01). Meanwhile, Fig. 15 shows no significant differences in the expression levels of MHC class II (I-A/I-E) (Fig. 15A) and CD86 (Fig. 15B) on pDC in the submandibular lymph nodes.

In Fig. 16, the ratio of pDC to lymphocytes in the nasal mucosa was significantly increased in the Low Volume administration group (Low Volume (+) group) compared to the Low Volume control group (Low Volume (-) group) (p < 0.05) (Fig. 16A (solid line box) and Fig. 16B (left)). By contrast, Fig. 17A shows no significant differences in the expression levels of MHC class II (I-A/I-E) and CD86 on pDC in the nasal mucosa.

In Fig. 16, the ratio of CD11c⁺Siglec-H⁺ cells to lymphocytes in the nasal mucosa was also significantly increased in the Low Volume administration group (Low Volume (+) group) compared to the Low Volume control group (Low Volume (-) group) (p < 0.01) (Fig. 16A (dashed line box) and Fig. 16B (right)). By contrast, Fig. 17B shows no significant differences in the expression levels of MHC class II (I-A/I-E) and CD86 on CD11c⁺Siglec-H⁺ cells in the nasal mucosa.

### Example 8: Study on immunostimulatory effect by intranasal administration (3)

In Example 8, the immunostimulatory effect of administration of the lactic acid bacterium was further studied using nasal mucosa cells collected from the nasal mucosa excised by dissection in Example 6.

### (1) Method

The nasal mucosa excised by dissection in Example 6 was temporarily stored in RPMI+ medium; the nasal mucosa was then scraped with tweezers in HBSS buffer solution; the resulting whole solution was passed through a 70-µm cell strainer and centrifuged (at 1,500 rpm and 4°C for 5 minutes); and the supernatant was removed and RPMI+ was then added to suspend the cell pellet. Next, the cells were counted, prepared at 6 × 10⁵ cells/ml, and seeded onto a 48-well plate at 500 µl/well. The culture medium was then added with no additive, ODN1585 (Invivogen) as CpG-A at 1 µM, or inactivated influenza virus H1N1 (HyTest) (H1N1) at 5 µg/ml in each well, and the mixture was incubated in a CO₂ incubator for 24 hours. After the end of incubation, the cell-containing medium was collected and centrifuged (at 5,000 rpm and 4°C for 2 minutes), and the culture supernatant was collected. The IFN-α level in the culture supernatant was measured using the VeriKine Interferon α ELISA Kit, Mouse (PBL Assay Science), the IFN-β level was measured using the VeriKine Interferon β ELISA Kit (PBL Assay Science), and IFN-λ level was measured using the Mouse IL-28B/IFN-lambda3 DuoSet ELISA (R&D systems), respectively.

### (2) Results

The results are as shown in Figs. 18 and 19. In Fig. 18, the nasal mucosa cells collected from the Low Volume administration group (Low Volume (+) group) showed a significantly higher level of IFN-α production in the case of CpG-A addition (CpG-A 1 µM) than the Low Volume control group (Low Volume (-) group) (p < 0.01). By contrast, no IFN-α production was detected in the case without any CpG-A addition (CpG-A 0 µM). These results have indicated that administration of the lactic acid bacterium induces IFN-α production after infection with pathogens such as bacteria and/or viruses, thereby producing an infectious disease preventive effect.

In Fig. 19, the nasal mucosa cells collected from the Low Volume administration group (Low Volume (+) group) tended to produce more IFN-α, IFN-β, and IFN-y in the case of addition of inactivated H1N1 (H1N1 5 µg/ml) than the nasal mucosa cells collected from the Low Volume control group (Low Volume (-) group). By contrast, no IFN-α production was detected in the case without any inactivated H1N1 addition (H1N1 0 µg/ml). These results have indicated that administration of the lactic acid bacterium induces IFN-α, IFN-β, and IFN-λ production after infection with the influenza virus, thereby producing an infectious disease preventive effect.

### Reference Example: Study on effect of preventing SARS-CoV-2 infection by oral administration

In Reference Example, the effect of the lactic acid bacterium on prevention of SARS-CoV-2 infection by oral administration, namely a publicly known administration method, was studied.

### (1) Method

### A. Upper respiratory tract viral inoculation study group

BALB/c mice (20 weeks old, female, body weight: about 20 g) were divided into 2 groups: a control group (standard diet intake group) and a test diet intake group (n=8 per group), and were reared, from 14 days before infection with SARS-CoV-2 to 2 or 3 days after infection, with free access to standard diet or test diet (containing 0.029 mass% of heat-killed *Lactococcus lactis* subsp. *lactis* JCM5805 strain as a lactic acid bacterium). Mice in each group were infected, under anesthesia on day 0 of infection, with the mouse-adapted SARS-CoV-2 QHmusX strain (Iwata-Yoshikawa N et al., Science Advances, (2022) 8(1): eabh3827) established at the National Institute of Infectious Diseases (NIID) at 5 × LD₅₀ (1.8 × 10⁴ TCID₅₀) (5 µl). Then, 2 or 3 days after infection, the mice were dissected and nasal-associated lymphoid tissues (NALT) were collected.

### B. Lower respiratory tract viral inoculation study group

BALB/c mice (21 weeks old, female, body weight: about 20 g) were divided into 2 groups: a control group (standard diet intake group) and a test food intake group (n=8 per group), and were reared, from 14 days before infection with SARS-CoV-2 to 2 or 3 days after infection, with free access (by oral administration) to standard diet or test diet (containing 0.05 mass% of heat-killed *Lactococcus lactis* subsp. *lactis* JCM5805 strain as a lactic acid bacterium). Mice in each group were infected, under anesthesia on day 0 of infection, with the mouse-adapted SARS-CoV-2 QHmusX strain (Iwata-Yoshikawa N et al., Science Advances, (2022) 8(1): eabh3827) established at the National Institute of Infectious Diseases (NIID) at 10 × LD₅₀ (3.5 × 10⁴ TCID₅₀) (30 µl). The mice were then dissected 3 days after infection and the lungs were collected.

### C. To extract and prepare RNA

RNA was extracted and prepared from the collected NALT and lungs in the same manner as described in Example 1(1)B.

### D. To quantify RNA

The RNA was quantified as described in Example 1(1)C.

### (2) Results

The results for the upper respiratory tract viral inoculation study group ((1) Method A) and the results for the lower respiratory tract viral inoculation study group ((1) Method B) are as shown in Fig. 20 and Fig. 21, respectively. In the upper respiratory tract viral inoculation study group, mice were fed with free access (by oral administration) to the standard diet or test diet from 14 days before to 2 or 3 days after infection with SARS-CoV-2 (for a total of 16 or 17 days), and as a result, the average daily intake of 2.38 g per mouse (1.19 mg of the lactic acid bacterium as the test diet) was found. Meanwhile, in the lower respiratory tract viral inoculation study group, mice were fed with free access (by oral administration) to the standard diet or test diet from 14 days before to 3 days after infection with SARS-CoV-2 (for a total of 17 days), and as a result, the average daily intake of 2.2 g per mouse (0.64 mg of the lactic acid bacterium as the test diet) was found.

In Fig. 20, the level of SARS-CoV-2 RNA in the NALT at 2 or 3 days after SARS-CoV-2 infection (approximately 48 or 72 hours after infection) was found to be lower in the lactic acid bacterium administration group than the control group.

In Fig. 21, the level of SARS-CoV-2 RNA in the NALT at 3 days after SARS-CoV-2 infection (approximately 72 hours after infection) was found to be lower in the lactic acid bacterium administration group than the control group.

## Claims

1. A pharmaceutical composition for topical administration, comprising a lactic acid bacterium as an active ingredient.

2. The pharmaceutical composition according to claim 1, wherein the topical administration is intranasal administration, sublingual administration, or inhalation administration.

3. The pharmaceutical composition according to claim 1, wherein the topical administration is administration to an upper and/or lower respiratory tract.

4. The pharmaceutical composition according to claim 3, wherein the upper respiratory tract is a nasal cavity.

5. The pharmaceutical composition according to claim 1 or 2 for inducing production of an interferon (IFN) by plasmacytoid dendritic cells in an upper and/or lower respiratory tract.

6. The pharmaceutical composition according to claim 5, wherein after a subject of administration of the pharmaceutical composition contracts an infectious disease, the production of IFN is induced in a subject.

7. The pharmaceutical composition according to claim 1 or 2 for prevention or treatment of an infectious disease or for use in reducing a risk of contracting an infectious disease in a subject of administration.

8. The pharmaceutical composition according to claim 7, wherein the infectious disease is a viral infectious disease.

9. The pharmaceutical composition according to claim 8, wherein the prevention of the viral infectious disease is prevention of onset of the viral infectious disease or prevention of aggravation of the viral infectious disease.

10. The pharmaceutical composition according to claim 8, wherein the viral infectious disease is COVID-19 or an influenza virus infection.

11. The pharmaceutical composition according to claim 8, wherein the viral infectious disease is an infection caused by a respiratory infection-causing virus.

12. The pharmaceutical composition according to claim 11, wherein the respiratory viral infection-causing virus is SARS-CoV-2 or an influenza virus.

13. The pharmaceutical composition according to claim 1 or 2, which is administered to a mammal.

14. The pharmaceutical composition according to claim 13, wherein the mammal is a human.

15. The pharmaceutical composition according to claim 1 or 2, wherein the number of doses is two or more.

16. The pharmaceutical composition according to claim 1 or 2, wherein a dosing interval is one day or more.

17. The pharmaceutical composition according to claim 8, wherein an effective period of the prevention of the viral infectious disease is 56 days from a last day of the topical administration.

18. The pharmaceutical composition according to claim 1 or 2, wherein a daily dose (based on 50 kg body weight of an adult) of the lactic acid bacterium is 1 mg or more and 1000 mg or less.

19. The pharmaceutical composition according to claim 1 or 2, wherein the lactic acid bacterium is *Lactococcus lactis* subsp. *lactis* JCM5805 strain.

20. A composition for topical administration, comprising *Lactococcus lactis* subsp. *lactis* as an active ingredient.

21. The composition for topical administration according to claim 20, which is a pharmaceutical composition.

22. The pharmaceutical composition according to claim 8, which is topically administered at a site of viral infection.

23. An immunostimulating composition for topical administration, comprising a lactic acid bacterium as an active ingredient.

24. The immunostimulating composition according to claim 22 or 23 for enhancing expression of IFN-inducible antiviral gene (ISG) in a submandibular lymph node and/or a spleen.

25. The immunostimulating composition according to claim 24, wherein the ISG is one or two or more kinds selected from the group consisting of Viperin, Isg15, and Mx1.

26. The immunostimulating composition according to claim 22 or 23 for increasing a ratio of pDC to lymphocytes in a spleen and/or a ratio of pDC to lymphocytes in nasal mucosa.

27. The immunostimulating composition according to claim 22 or 23 for increasing a ratio of CD11b⁺Siglec-H⁺ cells to lymphocytes in nasal mucosa.

28. A method of preventing or a method of treating an infectious disease, a method of reducing a risk of contracting an infectious disease, or a method of immunostimulation, the method comprising the step of topically administering an effective amount of lactic acid bacterium or a composition comprising the lactic acid bacterium to a subject in need thereof.

29. Use of a lactic acid bacterium for the manufacture of an infectious disease prophylactic or therapeutic agent for topical administration, for the manufacture of an infectious disease contraction risk reducing agent for topical administration, or for the manufacture of an immunostimulating agent for topical administration, or as an infectious disease prophylactic or therapeutic agent for topical administration, as an infectious disease contraction risk reducing agent for topical administration or an immunostimulating agent for topical administration, or in a method of preventing or treating an infectious disease by topical administration, in a method of reducing a risk of contracting an infectious disease by topical administration, or in a method of immunostimulation by topical administration.

30. A lactic acid bacterium for use in prevention or treatment of an infectious disease by topical administration, for use in reducing a risk of contracting an infectious disease by topical administration, or for use in immunostimulation by topical administration.
